(19) Europäisches Patentamt
European Patent Office
Office européen des brevets

(11) **EP 3 529 621 B1**

(12) **EUROPEAN PATENT SPECIFICATION**

(45) Date of publication and mention
of the grant of the patent:
**04.03.2020 Bulletin 2020/10**

(51) Int Cl.:
*C12Q 1/6841* (2018.01)     *G01N 33/574* (2006.01)
*C12N 5/078* (2010.01)

(21) Application number: **17786930.2**

(22) Date of filing: **19.10.2017**

(86) International application number:
**PCT/EP2017/076784**

(87) International publication number:
**WO 2018/073381 (26.04.2018 Gazette 2018/17)**

(54) **METHOD FOR DETERMINING PROGNOSIS OF CANCER**

VERFAHREN ZUR BESTIMMUNG DER PROGNOSE VON KREBS

PROCÉDÉ DE DÉTERMINATION D'UN PRONOSTIC DE CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2016 GB 201617722**

(43) Date of publication of application:
**28.08.2019 Bulletin 2019/35**

(73) Proprietor: **Queen Mary University of London**
**London E1 4NS (GB)**

(72) Inventors:
• **GUO, Tianyu**
  **London E1 4NS (GB)**
• **XU, Lei**
  **London E1 4NS (GB)**
• **MAO, Xueying**
  **London E1 4NS (GB)**
• **LU, Yong-Jie**
  **London E1 4NS (GB)**

(74) Representative: **Leonard, Thomas Charles**
**Kilburn & Strode LLP**
**Lacon London**
**84 Theobalds Road**
**London WC1X 8NL (GB)**

(56) References cited:
• **HUME R; WEST J T; MALMGREN R A; CHU E A: "QUANTITATIVE OBSERVATIONS OF CIRCULATING MEGAKARYOCYTES IN THE BLOOD OF PATIENTS WITH CANCER.", THE NEW ENGLAND JOURNAL OF MEDICINE, vol. 270, no. 3, 16 January 1964 (1964-01-16), pages 111-117, XP009501616, ISSN: 0028-4793**
• **ALEXANDER ZASLAVSKY ET AL: "Platelet-derived thrombospondin-1 is a critical negative regulator and potential biomarker of angiogenesis", BLOOD, vol. 115, 19 January 2010 (2010-01-19), pages 4605-4613, XP055427409, US ISSN: 0006-4971, DOI: 10.1182/blood-2009-09-242065**
• **M. A. LEVERSHA ET AL: "Fluorescence In situ Hybridization Analysis of Circulating Tumor Cells in Metastatic Prostate Cancer", CLINICAL CANCER RESEARCH, vol. 15, no. 6, 1 January 2009 (2009-01-01), pages 2091-2097, XP055020139, ISSN: 1078-0432, DOI: 10.1158/1078-0432.CCR-08-2036**
• **LEI XU ET AL: "Optimization and Evaluation of a Novel Size Based Circulating Tumor Cell Isolation System", PLOS ONE, vol. 10, no. 9, 23 September 2015 (2015-09-23), page e0138032, XP055428114, DOI: 10.1371/journal.pone.0138032**
• **XU LEI ET AL: "Analysis of prostate cancer circulating tumour cells isolated with a novel size-based system", THE LANCET, vol. 387, 25 February 2016 (2016-02-25), page S25, XP029433895, ISSN: 0140-6736, DOI: 10.1016/S0140-6736(16)00412-8**

**(Cont. next page)**

**EP 3 529 621 B1**

- **GUILIN TANG ET AL: "High-level CD34 expression on megakaryocytes independently predicts an adverse outcome in patients with myelodysplastic syndromes", LEUKEMIA RESEARCH, NEW YORK,NY, US, vol. 35, no. 6, 13 January 2011 (2011-01-13), pages 766-770, XP028383556, ISSN: 0145-2126, DOI: 10.1016/J.LEUKRES.2011.01.009 [retrieved on 2011-01-19]**

- **LEI XU ET AL: "The Novel Association of Circulating Tumor Cells and Circulating Megakaryocytes with Prostate Cancer Prognosis", CLINICAL CANCER RESEARCH, vol. 23, no. 17, 14 June 2017 (2017-06-14) , pages 5112-5122, XP055424950, US ISSN: 1078-0432, DOI: 10.1158/1078-0432.CCR-16-3081**

**Description**

The present application relates to methods for determining the prognosis of subjects with prostate cancer, including prostate cancer (PCa). The methods use circulating megakaryocytes.

Prostate cancer is the most common cancer and the second most frequent cause of cancer-related death in European and North American men. Metastasis is the main culprit of death in patients with localized cancer and hormone-resistance is the main cause for treatment failure. The molecular mechanisms which determine the aggressiveness and progression potential of prostate cancer are not clear yet. Prostate specific antigen (PSA) is the most widely used biomarker at present. However, when a metastasized disease progresses to castration-resistant prostate cancer (CRPC), PSA is unsatisfactory for disease monitoring. For instance, a flare in PSA levels occurs in many patients who respond to alternative 2nd line hormone therapy or chemotherapy.

Therefore, when a metastasized disease progresses to castration-resistant prostate cancer (CRPC), there is currently a dearth of clinically useful biomarkers for prognosis prediction and therapy response monitoring. Accordingly, there is a need to identify novel biomarkers which can monitor disease status precisely, accessibly and in real time.

Cancer is a heterogeneous disease and cancer cells evolve during disease progression and in response to treatment, influencing the sensitivity of cancer cells to treatment. Therefore, frequent tumor sampling to temporarily detect the molecular and cellular features of a cancer is required for precise therapeutic interventions. This is practically difficult using tissue biopsies due to their invasive nature and the cost of acquiring diagnostic samples.

Diagnostic and prognostic methods based on patient blood samples are therefore desirable. The desirability of such methods is not restricted to prostate cancer. Such methods may include using CTCs. Current CTC technologies, such as CellSearch® (the only FDA approved system), mainly rely on the capture and identification of those CTCs that express epithelial phenotype-specific markers epithelial cell adhesion molecule (EpCAM) and cytokeratin (CK). However, CellSearch® has been reported to detect fewer than two CTCs in many metastatic prostate cancer patients (Scher et al., Lancet Oncol. 10, 233-239 (2009)). A size- and/or deformability-based CTC isolation system, Parsortix™, has been optimized to capture CTCs based on the much larger size of tumor cells than the surrounding blood cells. Parsortix™ is described in EP2790020, which relates to methods and apparatus for segregating circulating tumor cells from blood cells in a whole blood sample. The efficiency of Parsortix™ in harvesting epithelial CTCs has been compared to Cell-Search® and IsoFlux, as well as its ability in isolation of CD45 negative (CD45-) circulating cells with mesenchymal properties Xu et al., PLoS One 10, e0138032 (2015). However, it is not presently possible to confirm the malignancy of CTCs whilst simultaneously confirming their identity using a marker based system. Moreover, Xu *et al.,* (2015) does not refer to megakaryocytes.

Leversha et al. Clin. Cancer Res., 15(6):2091-2097 (2009) discuss fluorescence *in situ* hybridization analysis of circulating tumor cells in metastatic prostate cancer. However, megakaryocytes and derivatives thereof were deliberately excluded from the authors' analysis. Hume et al., N. Engl. J. Med., 270:111-7 (1964) describes quantitative observations of circulating megakaryocytes in the blood of patients with cancer, and no relationship between numbers of circulating megakaryocytes and prognosis was demonstrated.

There remains in the art the need for a reliable and accurate method for the prognosis of cancer.

<u>Summary of the invention</u>

To further investigate CTCs with mesenchymal features, the inventors have developed a novel technique to perform multiple rounds of fluorescence *in situ* hybridization (FISH) on the same slides after immunofluorescence staining in order to simultaneously identify epithelial and mesenchymal cell features and changes at multiple genomic regions. Use of this technique allows confirmation of the malignancy of CTCs with a mesenchymal phenotype. The role of mesenchymal CTCs in risk prediction has also been determined for either newly diagnosed or progressed prostate cancer patients as well as their role in monitoring of therapy response.

The inventors have also unexpectedly identified an increase of circulating megakaryocytes (MK, also known as megacaryocytes) in blood samples from prostate cancer patients, which were related to prognosis in patients with progressive castration resistant prostate cancer (CRPC). Megakaryocytes have previously been considered irrelevant to cancer diagnosis, for example in a study aiming to further characterize CTCs for use as a biomarker in CRPC, in which the megakaryocytes were excluded from the analysis (Leversha et al., Clin Cancer Res, 15(6): 2091-2097 (2009)). Li et al., Journal of Bone and Mineral Research, 26(1): 125-134 (2011) describe local inhibitory effects of megakaryocytic cells in prostate cancer skeletal metastasis which occurs in the bone. Li *et al.,* (2011) does not disclose the entry of megakaryocytes into the bloodstream.

In a first aspect of the invention there is provided a method for determining the prognosis of prostate cancer in a subject. The method comprises measuring the amount of megakaryocytes in a blood sample from the subject.

The present disclosure also provides a method of treating cancer in a subject whose prognosis has been determined according to a method of the first aspect of the invention. The method further comprises administering a therapeutic agent to the subject and/or adopting a therapeutic regimen.

The present disclosure also provides an isolated mixture of megakaryocytes and CTCs.

The present disclosure also provides megakaryocytes for use in the diagnosis of cancer.

The present disclosure also provides megakaryocytes for use in the treatment of cancer.

The present disclosure also provides a method of treating cancer comprising administration of megakaryocytes.

The present disclosure also provides the use of circulating megakaryocytes isolated from a subject in an *ex vivo* and/or *in vitro* method of diagnosing or determining prognosis of cancer.

The present disclosure also provides a stripping buffer comprising 1 % to 3 % Sodium dodecyl sulfate (SDS), 0.055 M to 0.075 M Tris-HCl and 0.5 % to 1.1 % β-mercaptoethanol and wherein the stripping buffer has a pH from 6.6 to 7.0. The stripping buffer may comprise 2 % SDS, 0.0625 M Tris-HCl and 0.8 % β-mercaptoethanol, wherein the stripping buffer has a pH of 6.8

The present disclosure also provides a kit comprising specific binding molecules which bind to one or more of CD34, CD41, CD45, CK and/or vimentin (VIM).

Reference is made to a number of Figures as follows:

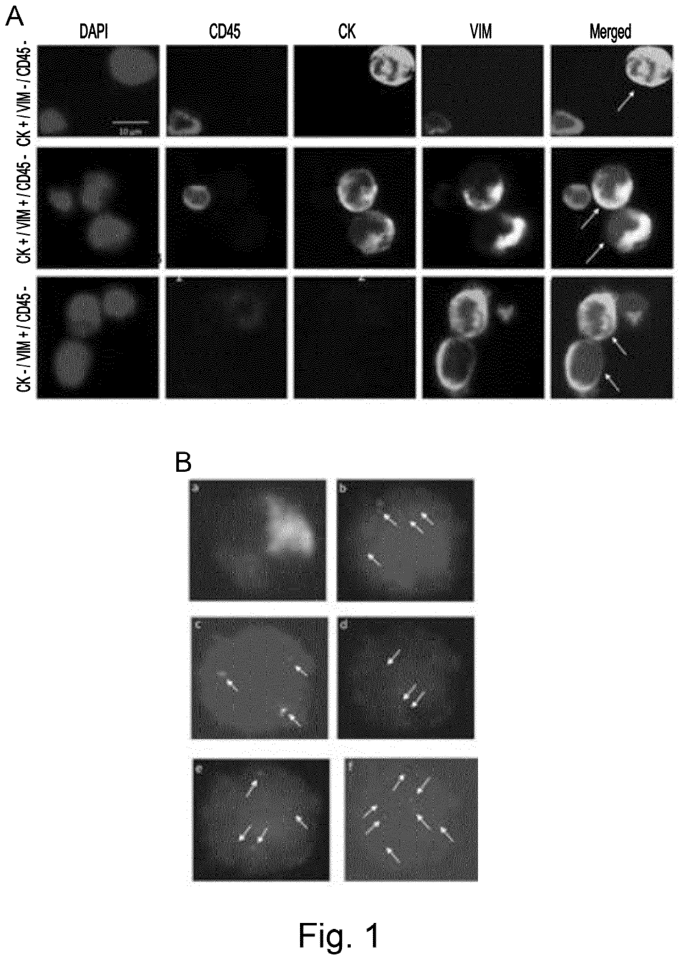

Figure 1 - Representative images for different populations of detected cells in prostate cancer patients following five rounds of FISH in a CTC. (A) Immunofluorescence image for three types of circulating cells. Top row: One CK+/VIM-/CD45- cell (epithelial type, arrowed) adjacent to one CD45+ lymphocyte. Middle row: Two CK+/VIM+/CD45- cells (undergoing epithelial to mesenchymal transition (EMTing type), arrowed) adjacent to one CD45+ lymphocyte. Bottom row: Two CK-/VIM+/CD45- cells (mesenchymal type, arrowed) adjacent to one CD45+ lymphocyte. (B) Image following five rounds of FISH on one CTC post-immunostaining. (a): a CK-/VIM+/CD45- circulating cell. (b): first round of FISH: 6q16 (upper left arrow) and AR (remaining three arrows). (c): second round FISH for ERG rearrangement: RP11-476D17 (upper right arrow) and RP11-95I21 (left most arrow); lower right arrow indicates substantially colocalised RP11-476D17 and RP11-95I21. (d): third round FISH: C-MYC (upper left and lower right arrows) and NKX3.1 (middle arrow between upper left and lower right arrows). (e): fourth round FISH: RB1 (leftmost and rightmost arrows) and PTEN (top-centre and bottom-centre arrows). (f): fifth round FISH: CCND1 (arrows pointing towards top-left) and 16q22.1 (arrows pointing to top-right and bottom-left). FISH signals are indicated by arrows as described.

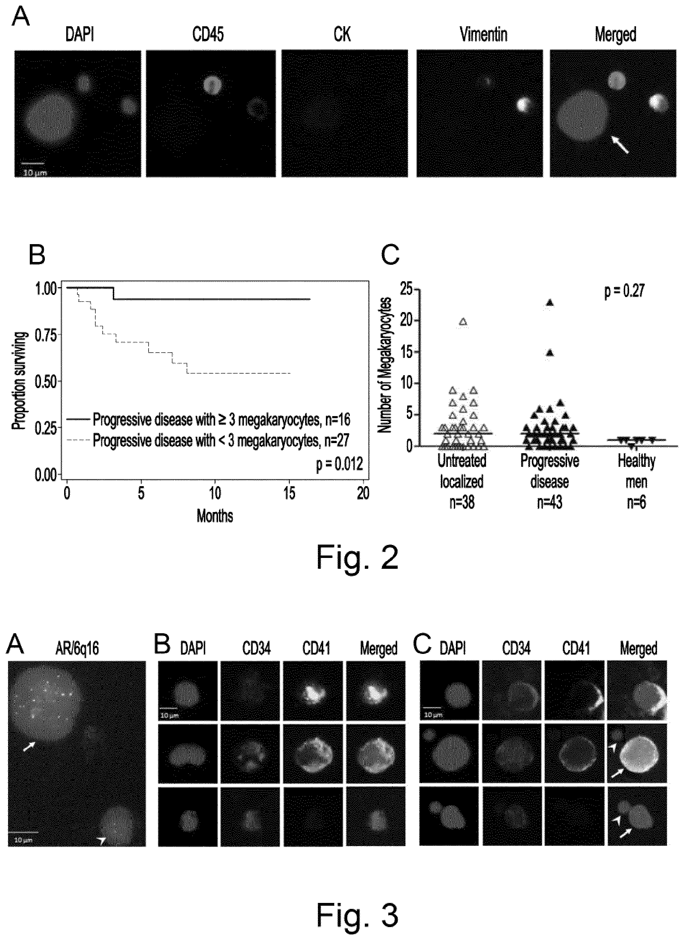

Figure 2 - Representative images and enumeration of megakaryocytes. (A) Example of a megakaryocyte (arrowed) with big and bright nucleus and negative for CD45, CK and VIM and two adjacent two CD45+/VIM+ lymphocytes. (B) Kaplan-Meier curve for overall survival showed progressive prostate cancer patients with less than 3 megakaryocytes had a significantly shorter survival rates (p = 0.012). (C) The median number of megakaryocytes in untreated localized disease was 2 cells/7.5 mL, with upper and lower quartiles of 0 and 4.25 cells/7.5 mL. The median number of megakaryocytes in 43 progressive disease was 2 cells/7.5 mL, with upper and lower quartiles of 1 and 3 cells/7.5 mL. The median number of megakaryocytes in six healthy controls was 1 cell/7.5 mL, with upper and lower quartiles of 1 and 1 cell/7.5 mL. There was no significant difference among the three groups (p = 0.27, Kruskal-Wallis test).

Figure 3 - FISH analysis and immunofluorescence to identify the nature of megakaryocytes. (A) FISH analysis by probe of AR (red) and 6q16 (green) showed polyploidy of the megakaryocyte (arrowed) and diploid of an adjacent lymphocyte (with arrow-head) which was CD45 positive in previous immunostaining. (B) PMA-treated K562 cell lines were positive for CD34 and CD41 staining with various patterns: strong CD41 but weak CD34 (upper row), similar signal strength of CD41 and CD34 (middle row), and strong CD34 but negative CD41 (lower row). Cells with obvious CD41 signals were larger than those without or with very weak CD41 staining. (C) Upper and middle row: megakaryocytes were positive for both CD41 and CD34, and signals of CD41 were relatively stronger. Lower row: a megakaryocyte was positive for CD34 but not CD41. Similarly, cells positive for CD41 were relatively larger. Adjacent CD45+ (image now shown) lymphocytes (with arrow-head) were negative for either CD34 or CD41.

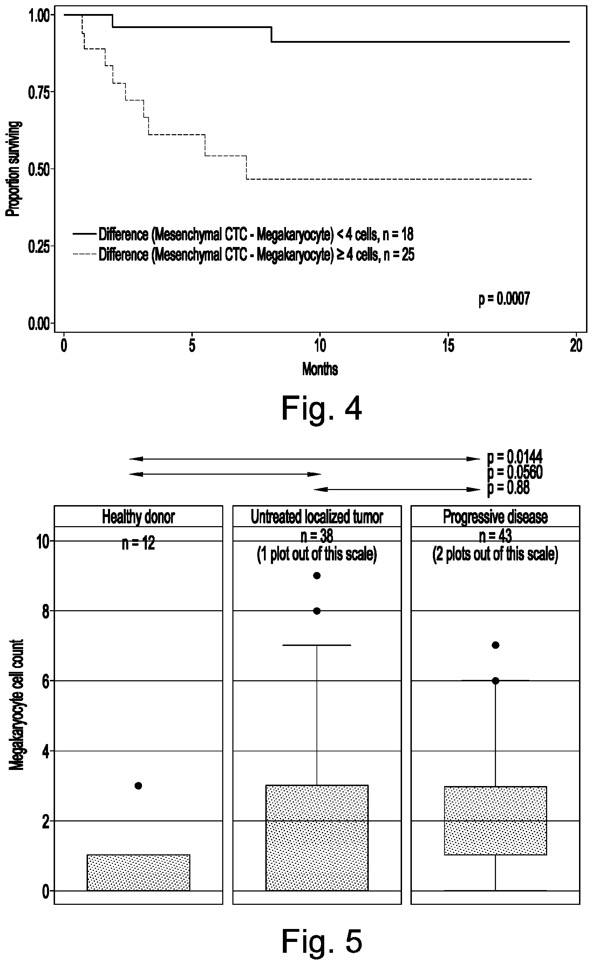

Figure 4 - Kaplan-Meier curve for overall survival showed progressive prostate cancer patients with number difference between mesenchymal CTCs and megakaryocytes of no less than four had even poorer survival (p = 0.0007).

Figure 5 - Box plots of megakaryocytes cell counts in healthy men, patients with untreated localized tumor, and with progressive disease. Median (IQR) value of megakaryocyte cell count were 1 (1-0), 2 (4.25-0), and 2 (3-1) for 12 healthy men, 38 patients with untreated localized tumor and 43 CRPC patients, respectively. One plot (20) from patients with untreated localized tumor and two plots (15 and 23) from patients with progressive disease were not shown in the figure due to out of above chosen scale.

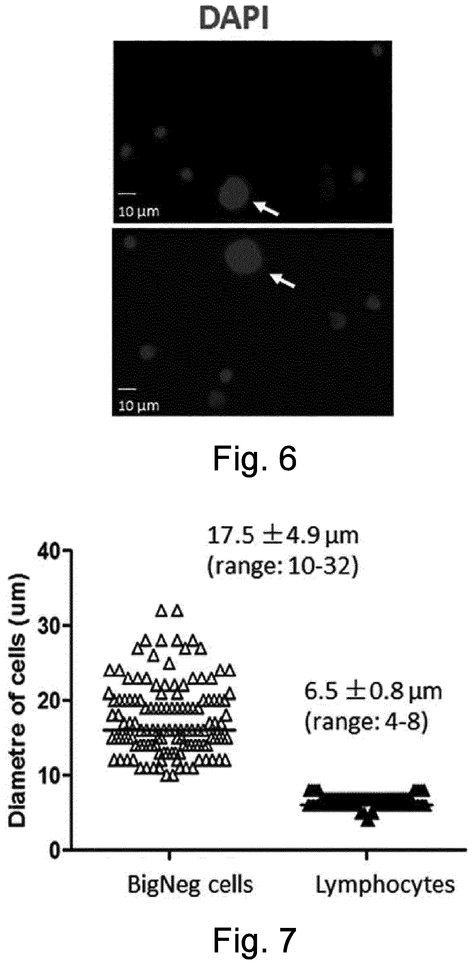

Figure 6 - Representative immunofluorescence/FISH images and nuclear size of megakaryocytes. Megakaryocytes (arrowed) could be easily screened and identified under low resolution image based on nucleus size alone.

Figure 7 - Comparison of nucleus diameter between megakaryocytes and lymphocytes where no size overlap was observed.

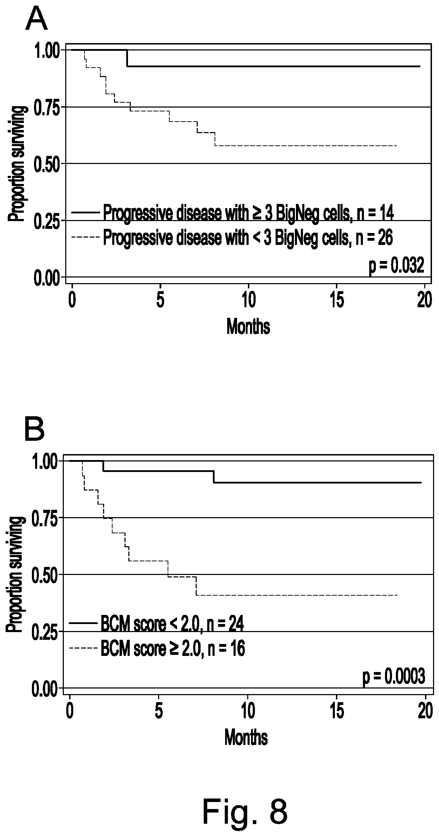

Figure 8 - Megakaryocyte cell count was associated with survival, and their megakaryocyte nature was confirmed by immunofluorescence. A, Kaplan-Meier curve for overall survival showed progressive prostate cancer patients with less than three megakaryocytes had significantly shorter survival rates (P ¼ 0.032). B, Kaplan-Meier curve for overall survival showed progressive prostate cancer patients with CMS 2.0 had even poorer survival (P ¼ 0.0003).

Detailed description

The present invention provides a method for determining the prognosis of prostate cancer in a subject. The method comprises measuring the amount of megakaryocytes in a blood sample from the subject. In some embodiments, if a poor prognosis is diagnosed or suspected, the methods of the invention may further comprise treating the patient for the prostate cancer.

Megakaryocytes

A megakaryocyte is a large bone marrow cell with a lobulated nucleus responsible for the production of blood thrombocytes (platelets), which are necessary for normal blood clotting. Megakaryocytes may be CD34 positive, CD41 positive, CD45 negative, CK negative and/or vimentin negative. For example, megakaryocytes may be CD45 negative, CK negative and vimentin negative. Megakaryocytes may also be CD34 positive and/or CD41 positive, CD45 negative, CK negative and vimentin negative. Megakaryocytes may also be CD34 positive, CD41 positive, CD45 negative, CK negative and vimentin negative. CD41 may be considered a marker for mature megakaryocytes. The data in the present application indicate that around 92% of megakaryocytes may be positive for CD41.

CD34 is an early development biomarker for megakaryocytes, known to the person skilled in the art. Megakaryocytes may therefore be CD34 positive cells.

CD41, also known as platelet glycoprotein IIb is known by the skilled person to be expressed by megakaryocytes. Megakaryocytes may therefore be CD41 positive cells.

CD45 is expressed by leukocytes. Megakaryocytes may therefore be CD45 negative cells.

CK is an epithelial marker which may be expressed by epithelial CTCs. Megakaryocytes may therefore be CK negative cells.

Vimentin is a mesenchymal marker which may be expressed by mesenchymal CTCs. Megakaryocytes may therefore be vimentin negative cells.

Megakaryocytes may also have a distinctively large nucleus. The nucleus of a megakaryocyte may have a dimension that is greater than or equal to 10 $\mu$m. The nuclear dimension of a megakaryocyte may be between 10 $\mu$m and 50 $\mu$m. The nuclear dimension of a megakaryocyte may be between 10 $\mu$m and 32 $\mu$m. For example, the nuclear dimension may therefore be greater than 11, 12, 13, 14, 15, 16, 17, 18, 19, 20, 25, or 30 $\mu$m. The average nuclear dimension of a megakaryocyte may be around 18 $\mu$m. For instance, the mean nuclear dimension of a megakaryocyte may be 17.8 $\pm$ 5.0 $\mu$m (range: 10 - 32 $\mu$m). In contrast, the mean nuclear dimension for lymphocytes may be 6.9 $\pm$ 1.0 $\mu$m (range: 4 - 9 $\mu$m). Typically the nuclear dimension is measured at the widest point in any cross section. A nuclear cross section may not be perfectly circular. Nevertheless, it will be understood that a nuclear dimension may be referred to as a nuclear diameter.

Megakaryocytes may also be polyploid. For example, megakaryocytes may be at least tetraploid (4N), octaploid or octoploid (8N), 16-ploid (16N), 32-ploid (32N) or 64-ploid (64N). As known by the person skilled in the art, "polyploid" cells and organisms are those containing more than two paired (homologous) sets of chromosomes.

Any of the above features of megakaryocytes may be combined in any combination. Cells defined herein as megakaryocytes which possess any of the features described herein may simply be referred to as "cells characterised by [any of the above features of megakaryocytes combined in any combination]".

Megakaryocytes may therefore be CD45 negative, CK negative and vimentin negative, have a nucleus with a dimension that is greater than or equal to 10 $\mu$m and are polyploid.

Megakaryocytes may also be CD34 positive, and/or CD41 positive, CD45 negative, CK negative and vimentin negative, have a nucleus with a dimension that is greater than or equal to 10 $\mu$m and are polyploid.

In some embodiments, less than or equal to a threshold amount of megakaryocytes indicates a poor prognosis. Alternatively, less than a threshold amount of megakaryocytes may indicate a poor prognosis. The threshold amount may be a suitable value on which to establish prognosis. The threshold amount may be a predetermined amount of megakaryocytes in a blood sample which has been experimentally identified as providing a useful distinguishing point between subjects with a good prognosis and subjects with a bad prognosis. The data presented in the present application indicates that 3 megakaryocytes per 7.5 mL of sample may be a preferred threshold value where the different prognostic profiles begin to become evident. In one embodiment, less than 3 megakaryocytes per 7.5 mL of sample indicates a poor prognosis. In another embodiment, less than or equal to 3 megakaryocytes per 7.5 mL of sample indicates a poor prognosis. However, other threshold values may also be used, such as 1, 2, 4, 5 or 6 megakaryocytes per 7.5 mL of sample. It will be appreciated that the threshold values may scale proportionately when the volume of the sample changes. For example, 3 megakaryocytes per 7.5 mL of sample is equivalent to 6 megakaryocytes per 15 mL of sample.

The amount of megakaryocytes per sample may be an average (e.g. median, mean, mode). Therefore, non-integer threshold values are also possible. In some embodiments, any non-integer threshold value between 1 and 6 megakaryocytes per 7.5 mL of sample may be used. Non-integer threshold values may also scale proportionately when the volume of the sample changes. For instance, 2.5 megakaryocytes per 7.5 mL of sample is equivalent to 5 megakaryocytes per 15 mL of sample and 3.5 megakaryocytes per 7.5 mL of sample is equivalent to 7 megakaryocytes per 15 mL of sample.

## Circulating tumour cells (CTCs)

In some embodiments, the method of the invention further comprises measuring the amount of CTCs in the blood sample. CTCs are cells that have been shed into the vasculature or lymphatics from a primary tumour or other cancer lesions and are carried around the body in the circulation. CTCs thus constitute seeds for the subsequent growth of additional tumours (metastases) in distant organs. Although defined as CTCs in the present application it will be apparent that the CTCs in a blood sample are not, strictly speaking, circulating once the blood sample has been taken. Cells defined herein as CTCs which possess any of the features described herein may simply be referred to as "cells characterised by [any of the above features of CTCs combined in any combination]".

CTCs may be have an epithelial phenotype (epithelial CTCs) a mesenchymal phenotype (mesenchymal CTCs) and may also be partially transitioned between epithelial and mesenchymal phenotypes (epithelial-mesenchymal CTCs or CTCs undergoing epithelial to mesenchymal transition (EMT)). Accordingly, the CTCs may be partially transitioned from epithelial CTCs to mesenchymal CTCs.

In some embodiments the CTCs are CD45 negative, CK positive and/or vimentin positive. Accordingly, the CTCs may be CD45 negative, CK positive and vimentin positive. Typically CTCs which are CD45 negative, CK positive and vimentin positive are partially transitioned from epithelial CTCs to mesenchymal CTCs.

In some embodiments the CTCs are CD45 negative, CK negative and/or vimentin positive. Accordingly, the CTCs may be CD45 negative, CK negative and vimentin positive. Typically CTCs which are CD45 negative, CK negative and vimentin positive are mesenchymal CTCs.

In some embodiments the CTCs are CD45 negative, CK positive and/or vimentin negative. Accordingly, the CTCs may be CD45 negative, CK positive and vimentin negative. Typically CTCs which are CD45 negative, CK positive and vimentin negative are epithelial CTCs.

CD45 is expressed by leukocytes. CTCs may therefore be CD45 negative cells. Epithelial CTCs, CTCs that are partially transitioned from epithelial CTCs to mesenchymal CTCs and mesenchymal CTCs may each be CD45 negative cells.

CK is an epithelial marker which may be expressed by epithelial CTCs. CK is down-regulated during the epithelial to mesenchymal transition. Accordingly CTCs that are partially transitioned from epithelial CTCs to mesenchymal CTCs may express CK. Mesenchymal CTCs typically do not express CK. Mesenchymal CTCs may therefore be CK negative cells.

Vimentin is a mesenchymal marker which may be expressed by mesenchymal CTCs. Vimentin is up-regulated during the epithelial to mesenchymal transition. Accordingly CTCs that are partially transitioned from epithelial CTCs to mesenchymal CTCs may express vimentin. Epithelial CTCs typically do not express vimentin. Epithelial CTCs may therefore be vimentin negative cells.

In some embodiments, greater than or equal to a threshold amount of CTCs indicates a poor prognosis. Alternatively, greater than a threshold amount of CTCs may indicate a poor prognosis. The threshold amount may be a suitable value on which to establish prognosis. The threshold amount may be a predetermined amount of CTCs in a blood sample which has been experimentally identified as providing a useful distinguishing point between subjects with a good prognosis and subjects with a bad prognosis. The data presented in the present application indicate that 10 CTCs per 7.5 mL of sample may be a preferred threshold value where the different prognostic profiles begin to become evident. When only CTCs which are partially transitioned from epithelial CTCs to mesenchymal CTCs and mesenchymal CTCs are included (i.e. when epithelial CTCs are excluded) the data presented in the present application indicate that 7 CTCs per 7.5 mL of sample may be a preferred threshold value where the different prognostic profiles begin to become evident. When only mesenchymal CTCs are included (i.e. when epithelial CTCs and CTCs which are partially transitioned from epithelial CTCs to mesenchymal CTCs are excluded) the data presented in the present application indicate that 5 CTCs per 7.5 mL of sample may be a preferred threshold value where the different prognostic profiles begin to become evident. However, other threshold values may also be used, such as 1, 2, 3, 4, 5, 6, 7, 8, 9, 10, 11, 12, 13, 14, 15, 16, 17, 18, 19 or 20 CTCs per 7.5 mL of sample. It will be appreciated that the threshold values may scale proportionately when the volume of the sample changes. For example, 10 CTCs per 7.5 mL of sample is equivalent to 20 CTCs per 15 mL of sample.

In some embodiments, the threshold amount of CTCs per 7.5 mL of sample is selected from the group consisting of:

(a) 10 CTCs per 7.5 mL of sample, wherein the CTCs comprise epithelial CTCs, CTCs that are partially transitioned from epithelial CTCs to mesenchymal CTCs and mesenchymal CTCs,
(b) 7 CTCs per 7.5 mL of sample, wherein the CTCs comprise CTCs that are partially transitioned from epithelial CTCs to mesenchymal CTCs and mesenchymal CTCs, and
(c) 5 CTCs per 7.5 mL of sample, wherein the CTCs comprise mesenchymal CTCs.

In some embodiments, the threshold amount of CTCs per 7.5 mL of sample is selected from the group consisting of:

(a) 10 CTCs per 7.5 mL of sample, wherein the CTCs consist of epithelial CTCs, CTCs that are partially transitioned from epithelial CTCs to mesenchymal CTCs and mesenchymal CTCs,
(b) 7 CTCs per 7.5 mL of sample, wherein the CTCs consist of CTCs that are partially transitioned from epithelial CTCs to mesenchymal CTCs and mesenchymal CTCs, and
(c) 5 CTCs per 7.5 mL of sample, wherein the CTCs consist of mesenchymal CTCs.

In some embodiments, the threshold amount of CTCs per 7.5 mL of sample is selected from the group consisting of:

(a) 10 CTCs per 7.5 mL of sample,
(b) 7 CTCs per 7.5 mL of sample, wherein epithelial CTCs are excluded, and
(c) 5 CTCs per 7.5 mL of sample, wherein epithelial CTCs and CTCs that are partially transitioned from epithelial CTCs to mesenchymal CTCs are excluded.

In the embodiments above, the epithelial CTCs, CTCs that are partially transitioned from epithelial CTCs to mesenchymal CTCs and mesenchymal CTCs may be defined by the features of each phenotype described herein.

Accordingly, greater than 10, 7 or 5 CTCs per 7.5 mL of sample may indicate a poor prognosis. Alternative values may apply, so that 1, 2, 3, 4, 5, 6, 7, 8, 9, 10, 11, 12, 13, 14, 15, 16, 17, 18, 19 or 20 CTCs per 7.5 mL of sample may indicate a poor prognosis.

The amount of CTCs per sample may be an average (e.g. median, mean, mode). Therefore, non-integer threshold values are also possible. In some embodiments, any non-integer threshold value between 1 and 20 CTCs per 7.5 mL of sample may be used. Non-integer threshold values may also scale proportionately when the volume of the sample changes. For instance, 9.5 CTCs per 7.5 mL of sample is equivalent to 19 CTCs per 15 mL of sample and 10.5 CTCs per 7.5 mL of sample is equivalent to 21 CTCs per 15 mL of sample.

The step of measuring the amount of CTCs in a blood sample according to the method of the invention may involve measuring the amount of epithelial CTCs, mesenchymal CTCs and/or CTCs that are partially transitioned from epithelial CTCs to mesenchymal CTCs.

In one embodiment of the invention, there is a provided a method of prognosis of prostate cancer, and in particular castration-resistant prostate cancer (CRPC), comprising determining the number of megakaryocytes in a blood sample from a patient wherein less than 3 megakaryocytes per 7.5 mL of blood sample indicates a poor prognosis.

Comparing the amount of CTCs and megakaryocytes to each other

In some embodiments, the method of the invention further comprises comparing the amount of CTCs and megakaryocytes to each other. The comparison of the amount of CTCs to the amount of megakaryocytes may involve the amount of epithelial CTCs, mesenchymal CTCs and/or CTCs that are partially transitioned from epithelial CTCs to mesenchymal CTCs. Typically, the comparison involves calculating the difference between the amount of CTCs and the amount of megakaryocytes, for example by subtracting the amount of megakaryocytes from the amount CTCs.

The difference between the amount of CTCs and the amount of megakaryocytes may simply and intuitively be calculated as amount of CTCs - amount of megakaryocytes. However, in all aspects of the invention, the difference between the amount of CTCs and the amount of megakaryocytes may alternatively be calculated as the amount of CTCs divided by the amount of megakaryocytes. Therefore, the comparison may involve calculating the ratio or the fraction of the amount of CTCs and the amount of megakaryocytes. Accordingly, it will be appreciated that as used herein the term "difference" may be substituted with the term "ratio", "fraction" or "fold difference".

In all aspects of the invention, the difference between the amount of CTCs and the amount of megakaryocytes may alternatively be calculated as the amount of CTCs minus the square of the amount of megakaryocytes. Accordingly, the following formula may be used to calculate the difference:

Amount of CTCs – (amount of megakaryocytes x amount of megakaryocytes)

In some embodiments, greater than or equal to a threshold difference between the amount of CTCs and the amount of megakaryocytes indicates a poor prognosis. Alternatively, greater than a threshold difference between amount of CTCs and the amount of megakaryocytes may indicates a poor prognosis. The threshold amount may be a predetermined difference between the amount of amount of CTCs and the amount of megakaryocytes in a blood sample which has been experimentally identified as providing a useful distinguishing point between subjects with a good prognosis and subjects with a bad prognosis. The data presented in the present application indicate that a difference of 4 or more cells per 7.5 mL of sample may be a preferred threshold value where the different prognostic profiles begin to become evident. Accordingly, in some embodiments, a difference of 4 or more cells per 7.5 mL of sample between the amount of megakaryocytes and the amount of CTCs indicates a poor prognosis.

In some embodiments, the difference between the amount of mesenchymal CTCs and the amount of megakaryocytes is determined. Therefore, the following formula may be used to calculate the difference between the amount of CTCs and the amount of megakaryocytes:

Amount of mesenchymal CTCs – (amount of megakaryocytes x amount of megakaryocytes)

A still further alternative to calculate the different can be:

$$\frac{(\text{mesenchymal CTC count} - \text{megakaryocyte count})}{\text{megakaryocyte count}}$$

Since the amount of megakaryocytes in a sample may be zero, the amount of megakaryocytes may be recorded as one rather than zero. Accordingly, if no megakaryocytes are detected in a sample the difference between the amount of CTCs and the amount of megakaryocytes calculated using the above formula will be the amount of CTCs minus one. Use of the above formula may provide an improved hazard ratio (HR) per unit increase of a marker (for example as discussed in Heller et al., 2016, Clinical Cancer Research, 23:1967-1973). However, the choice of formula is not critical to conduct the invention.

Accordingly, the difference between the amount of CD45 negative, CK negative and/or vimentin positive CTCs and the amount of megakaryocytes may be determined. The difference between the amount of CD45 negative, CK negative and vimentin positive CTCs and the amount of megakaryocytes may be determined. The data presented in the present application particularly indicate that a difference of 4 or more cells per 7.5 mL of sample may be a preferred threshold value where the different prognostic profiles begin to become evident when the CTCs are mesenchymal CTCs and/or the CTCs are CD45 negative, CK negative and/or vimentin positive. Accordingly, in some embodiments, a difference of 4 or more cells per 7.5 mL of sample between the amount of megakaryocytes and the amount of mesenchymal CTCs and/or CD45 negative, CK negative and/or vimentin positive CTCs indicates a poor prognosis.

However, other threshold values may also be used, such as 1, 2, 4, 5, 6, 7, 8, 9, 10, 11, 12, 13, 14, 15, 16, 17, 18, 19 or 20 cells per 7.5 mL of sample. It will be appreciated that the threshold values may scale proportionately when the volume of the sample changes. For example, 4 cells per 7.5 mL of sample is equivalent to 8 cells per 15 mL of sample.

Non-integer threshold values of the difference between the amount of CTCs and the amount of megakaryocytes are also possible. In some embodiments, any non-integer threshold value between 1 and 20 cells per 7.5 mL of sample may be used. Non-integer threshold values may also scale proportionately when the volume of the sample changes. For instance, 3.5 cells per 7.5 mL of sample is equivalent to 7 cells per 15 mL of sample and 4.5 cells per 7.5 mL of sample is equivalent to 9 cells per 15 mL of sample.

Any threshold value for the difference between the amount of CTCs and the amount of megakaryocytes calculated by subtraction may alternatively be formulated as a ratio (the number of times the amount of CTCs contains or is contained within the amount of megakaryocytes) or a fraction or fold difference (calculated by dividing the amount of CTCs by the amount of megakaryocytes). For example, if a sample contains 7 CTCs and 3 megakaryocytes per 7.5 mL the difference (calculated by subtraction) between the amount of CTCs and the amount of megakaryocytes is 4, the ratio of CTCs to megakaryocytes is 7:3, the fraction is 7/3 and the fold difference is 2.33 (to three significant figures).

In some embodiments, the difference between the amount of mesenchymal CTCs and the amount of megakaryocytes is determined. Accordingly, the difference between the amount of CD45 negative, CK negative and/or vimentin positive CTCs and the amount of megakaryocytes may be determined. The difference between the amount of CD45 negative, CK negative and vimentin positive CTCs and the amount of megakaryocytes may be determined. The data presented in the present application particularly indicate that a difference of 4 or more cells per 7.5 mL of sample may be a preferred threshold value where the different prognostic profiles begin to become evident when the CTCs are mesenchymal CTCs and/or the CTCs are CD45 negative, CK negative and/or vimentin positive. Accordingly, in some embodiments, a difference of 4 or more cells per 7.5 mL of sample between the amount of megakaryocytes and the amount of mesenchymal CTCs and/or CD45 negative, CK negative and/or vimentin positive CTCs indicates a poor prognosis.

However, other threshold values may also be used, such as 1, 2, 4, 5, 6, 7, 8, 9, 10, 11, 12, 13, 14, 15, 16, 17, 18, 19 or 20 cells per 7.5 mL of sample. It will be appreciated that the threshold values may scale proportionately when the volume of the sample changes. For example, 4 cells per 7.5 mL of sample is equivalent to 8 cells per 15 mL of sample.

Non-integer threshold values of the difference between the amount of CTCs and the amount of megakaryocytes are also possible. In some embodiments, any non-integer threshold value between 1 and 20 cells per 7.5 mL of sample may be used. Non-integer threshold values may also scale proportionately when the volume of the sample changes. For instance, 3.5 cells per 7.5 mL of sample is equivalent to 7 cells per 15 mL of sample and 4.5 cells per 7.5 mL of sample is equivalent to 9 cells per 15 mL of sample.

In one embodiment of the invention, there is a provided a method of prognosis of prostate cancer, and in particular castration-resistant prostate cancer (CRPC), comprising measuring the amount of megakaryocytes and the amount of mesenchymal CTCs in the blood sample, wherein a combined risk score of greater than or equal to 2.0 indicates a poor prognosis, wherein the combined risk score is calculated according to the formula (mesenchymal CTC count - megakaryocyte count) / megakaryocyte count. The megakaryocytes can be CD34 positive and/or CD41 positive, CD45 negative, CK negative and vimentin negative.

Samples

The methods of the invention comprise measuring the amount of megakaryocytes in a blood sample from a subject. Optionally, the amount of CTCs in the blood sample may also be measured. The megakaryocytes and CTCs may be measured in different blood samples.

The term "blood sample" is used herein to refer to blood which originates from a subject and/or which has been isolated from a subject. The blood sample is typically obtained from a vein. The blood sample may be obtained by venupuncture or using a fingerstick. Typically, the blood sample is acquired at the middle of phlebotomy after the collection for routine clinical blood test to avoid contamination with epithelial cells from the skin.

The term "blood sample" may denote any suitable sample of blood known to the person skilled in the art, such as whole blood, blood plasma and blood serum. Typically, the amount of megakaryocytes and/or CTCs is determined in a whole blood sample. The amount of other analytes, such as PSA, may be determined in a blood serum sample. The blood serum may be separated by standard laboratory techniques known to the person skilled in the art.

The methods of the invention optionally comprise the step of obtaining a blood sample from the subject.

In some embodiments, the method comprises obtaining a first blood sample from the subject and obtaining a second blood sample from the subject. Therefore, in some embodiments the method comprises measuring the amount of megakaryocytes and/or CTCs in one or more blood samples from a subject.

In some embodiments, the method comprises measuring the amount of megakaryocytes in a first sample and measuring the amount of CTCs in a second sample. Accordingly, in some embodiments the CTCs and megakaryocytes are not analysed in the same blood sample. The first and second samples may be derived from the same initial sample but processed separately. Alternatively, the first and second samples may be obtained at different time points and locations. The analysis of CTCs and megakaryocytes may be run separately or concurrently, as appropriate.

Accordingly, the invention also provides a method of determining prognosis of prostate cancer in a subject comprising measuring the amount of megakaryocytes in a first sample from the subject and measuring the amount of CTCs in a second sample from the subject.

The methods of the invention may optionally comprise a step of comparing the amount of megakaryocytes and/or CTCs and/or the difference between the amount of CTCs and the amount of megakaryocytes with a control or reference.

The comparison with a control or reference may be to determine if prostate cancer is present or not. Said comparison step may also detect the presence of particular types of cancer (including CRPC) and/or determine how extensive the therapeutic intervention should be for each patient. For example, the methods of the invention may detect aggressive prostate cancer and distinguish this from less-aggressive prostate cancer.

Determining the severity of the therapeutic intervention required may be of particular importance in CRPC. Said comparison step may include comparing the amount of the megakaryocytes and/or CTCs and/or the difference between the amount of CTCs and the amount of megakaryocytes with the amount of a type of cells in a control or reference sample.

This control may be from the same sample, but be a cell type different from the cell type being assayed for the diagnosis or prognosis of prostate cancer (an internal reference).

The comparison step may therefore be with reference to the amount of other circulating cells within the same sample, which may include but are not restricted to red blood cells, platelets and white blood cells such as lymphocytes. Alternatively, the reference may be a biological sample taken from a healthy subject/individual. Alternatively still, the method may use reference data obtained from samples from the same patient at a previous point in time. In this way, the effectiveness of any treatment can be assessed and a prognosis for the patient determined. However, a control or reference sample is not always required.

In some embodiments, the method comprises enriching the sample for megakaryocytes and/or CTCs. The enriching may comprise selecting for, increasing the concentration of and/or isolating the megakaryocytes and/or CTCs. Accordingly, the enriching may be performed by isolating and/or counting the megakaryocytes and/or CTCs. The enriching, the isolating and/or the counting may be by size- and/or deformability-based sorting of the megakaryocytes and/or CTCs. The enriching may be performed according to any suitable method known to the skilled person, for example using Parsortix™ (such as a device according to EP2790020), Flowcytometry cell sorter, CellSearch® and/or IsoFlux.

In some embodiments, the size- and/or deformability-based sorting of the CTCs excludes any cells with a dimension below about 10 $\mu$m. Therefore, the dimension of a CTC may be greater than 11, 12, 13, 14, 15, 16, 17, 18, 19, 20, 25, or 30 $\mu$m. Size- and/or deformability-based separation modules and arrays are described below.

A sample (e.g. blood sample) can be enriched for the analytes or cells of interest (e.g. megakaryocytes and/or CTCs) using one or more any methods known in the art (e.g. Guetta, EM et al., Stem Cells Dev, 13(1):93-9 (2004)) or described herein. The enrichment increases the concentration of cells or ratio of cells of interest to other cells in the sample. For example, enrichment can increase concentration of an analyte or cell of interest, such as a megakaryocyte and/or a CTC, by a factor of at least 2, 4, 6, 8, 10, 20, 50, 100, 200, 500, 1,000, 2,000, 5,000, 10,000, 20,000, 50,000, 100,000, 200,000, 500,000, 1,000,000, 2,000,000, 5,000,000, 10,000,000, 20,000,000, 50,000,000, 100,000,000, 200,000,000, 500,000,000, 1,000,000,000, 2,000,000,000, or 5,000,000,000 fold over its concentration in the original sample. Enrichment can also increase concentration of cells in volume of cells / total volume of sample (removal of fluid). A fluid sample (e.g., a blood sample) of greater than 10, 15, 20, 50, or 100 mL total volume comprising components of interest, and it can be concentrated such that the rare component of interest into a concentrated solution of less than 0.01, 0.02, 0.05, 0.1, 0.2, 0.5, 1, 2, 3, 5, or 10 mL total volume.

Enrichment can occur using one or more types of separation modules. Several different modules are described herein, all of which can be fluidly coupled with one another in the series for enhanced performance.

Enrichment may alternatively occur by selective lysis.

Enrichment for cells of interest may occur using one or more size and deformability-based separation modules. Examples of size- and/or deformability-based separation modules include filtration modules, sieves, matrixes, etc. Examples of size- and/or deformability-based separation modules contemplated for use in the present invention include those disclosed in International Publication No. WO 2004/113877. Other size based separation modules are disclosed in International Publication No. WO 2004/0144651.

A size- and/or deformability-based separation module comprises one or more arrays of obstacles forming a network of gaps. The obstacles are configured to direct particles as they flow through the array/network of gaps into different directions or outlets based on the particle's hydrodynamic size and deformability. For example, as a blood sample flows through an array of obstacles, nucleated cells or low deformability cells having a hydrodynamic size larger than a predetermined size, e.g., 8 microns, are directed to a first outlet located on the same side or the opposite side of the array of obstacles from the fluid flow inlet, while the enucleated cells or cells having a hydrodynamic size smaller than a predetermined size, e.g., 8 microns or high deformability cells with a predetermined size, e.g., 10 microns are directed to a second outlet also located on the opposite side of the array of obstacles from the fluid flow inlet.

An array can be configured to separate cells smaller or larger than a predetermined size by adjusting the size of the gaps, obstacles, and offset in the period between each successive row of obstacles. For example, in some embodiments, obstacles or gaps between obstacles can be up to 10, 20, 50, 70, 100, 120, 150, 170, or 200 microns in length or about 2, 4, 6, 8, 9 or 10 microns in length. In some embodiments, an array for size- and/or deformability-based separation includes more than 100, 500, 1,000, 5,000, 10,000, 50,000 or 100,000 obstacles that are arranged into more than 10, 20, 50, 100, 200, 500, or 1000 rows. Preferably, obstacles in a first row of obstacles are offset from a previous (upstream) row of obstacles by up to 50% the period of the previous row of obstacles. In some embodiments, obstacles in a first row of obstacles are offset from a previous row of obstacles by up to 45, 40, 35, 30, 25, 20, 15 or 10% the period of the previous row of obstacles. Furthermore, the distance between a first row of obstacles and a second row of obstacles can be up to 10, 20, 50, 70, 100, 120, 150, 170 or 200 microns. A particular offset can be continuous (repeating for multiple rows) or non-continuous. In some embodiments, a separation module includes multiple discrete arrays of obstacles fluidly coupled such that they are in series with one another. Each array of obstacles has a continuous offset. But each subsequent (downstream) array of obstacles has an offset that is different from the previous (upstream) offset. Preferably, each subsequent array of obstacles has a smaller offset that the previous array of obstacles. This allows for a refinement in the separation process as cells migrate through the array of obstacles. Thus, a plurality of arrays can be fluidly coupled in series or in parallel, (e.g., more than 2, 4, 6, 8, 10, 20, 30, 40, 50). Fluidly coupling separation modules (e.g., arrays) in parallel allows for high-throughput analysis of the sample, such that at least 1, 2, 5, 10, 20, 50, 100, 200, or 500 mL per hour flows through the enrichment modules or at least 1, 5, 10, or 50 million cells per hour are sorted or flow through the device.

In an exemplary size- and/or deformability-based separation module, obstacles (which may be of any shape) are coupled to a flat substrate to form an array of gaps. A transparent cover or lid may be used to cover the array. The obstacles form a two-dimensional array with each successive row shifted horizontally with respect to the previous row of obstacles, where the array of obstacles directs component having a hydrodynamic size smaller than a predetermined size in a first direction and component having a hydrodynamic size larger that a predetermined size in a second direction. For enriching epithelial or circulating tumour cells from enucleated, the predetermined size of an array of obstacles can be get at 6-12 $\mu$m or 6-8 $\mu$m. For enriching megakaryocytes and/or CTCs from a mixed sample (e.g. a blood sample) the predetermined size of an array of obstacles can be between 6-12 $\mu$m, 8-10 $\mu$m or 9 $\mu$m. The flow of sample into the array of obstacles can be aligned at a small angle (flow angle) with respect to a line-of-sight of the array. Optionally, the array is coupled to an infusion pump to perfuse the sample through the obstacles. The flow conditions of a size- and/or deformability-based separation module are such that cells are sorted by the array with minimal damage. This allows for downstream analysis of intact cells to be more efficient and reliable.

The size- and/or deformability-based separation module may be a Parsortix™ system (such as a device according to EP2790020). The Parsortix™ system is available commercially as Parsortix PR1. The Parsortix™ system may be the optimised Parsortix™ system described in Xu *et al.*, (2015).

A size- and/or deformability-based separation module may comprise an array of obstacles configured to direct cells larger than a predetermined size to migrate along a line-of-sight within the array (e.g. towards a first outlet or bypass channel leading to a first outlet), while directing cells and analytes smaller than a predetermined size to migrate through the array of obstacles in a different direction than the larger cells (e.g. towards a second outlet).

A variety of enrichment protocols may be utilized, although gentle handling of the cells is preferred to reduce any mechanical damage to the cells or their DNA. This gentle handling may serve to preserve the small number of megakaryocytes and/or CTCs in the sample. Integrity of the cells being evaluated is an important feature to permit the distinction between the megakaryocytes and/or CTCs and other cells in the sample. In particular, the enrichment and separation of the megakaryocytes and/or CTCs using the arrays of obstacles produces gentle treatment which minimizes cellular damage and maximizes nucleic acid integrity permitting exceptional levels of separation and the ability to subsequently utilize various formats to very accurately analyse the genome of the cells which are present in the sample in extremely low numbers.

Enrichment for cells of interest (e.g. megakaryocytes and/or CTCs) occurs using one or more capture modules that selectively inhibit the mobility of one or more cells of interest. Preferably, a capture module is fluidly coupled downstream to a size- and/or deformability-based separation module. Capture modules can include a substrate having multiple obstacles that restrict the movement of cells or analytes greater than a predetermined size. Examples of capture modules that inhibit the migration of cells based on size are disclosed in U.S. Patent No. 5,837,115 and 6,692,952.

In some embodiments, a capture module includes a two dimensional array of obstacles that selectively filters or captures cells or analytes having a hydrodynamic size greater than a particular gap size (predetermined size), International Publication No. WO 2004/113877.

In some cases a capture module captures analytes (e.g., cells of interest or not of interest) based on their affinity. For example, an affinity-based separation module that can capture cells or analytes can include an array of obstacles adapted for permitting sample flow through, but for the fact that the obstacles are covered with binding moieties that selectively bind one or more analytes (e.g., cell populations) of interest (e.g., megakaryocytes and/or CTCs) or analytes not-of-interest (e.g., white blood cells, red blood cells or epithelial cells). Arrays of obstacles adapted for separation by capture can include obstacles having one or more shapes and can be arranged in a uniform or non-uniform order. A two-dimensional array of obstacles may be staggered such that each subsequent row of obstacles is offset from the previous row of obstacles to increase the number of interactions between the analytes being sorted (separated) and the obstacles.

Binding moieties coupled to the obstacles can include e.g., proteins (e.g., ligands/receptors), nucleic acids having complementary counterparts in retained analytes, antibodies, etc. In some embodiments, an affinity-based separation module comprises a two-dimensional array of obstacles covered with one or more antibodies selected from the group consisting of: anti-CD34, anti-CD41, anti-CD45, anti-CK and anti-vimentin. Examples of such affinity-based separation modules are described in International Publication No. WO 2004/029221.

A capture module may utilize a magnetic field to separate and/or enrich one or more analytes (cells) based on a magnetic property or magnetic potential in such analyte of interest or an analyte not of interest. For example, red blood cells which are slightly diamagnetic (repelled by magnetic field) in physiological conditions can be made paramagnetic (attributed by magnetic field) by deoxygenation of the haemoglobin into methaemoglobin. This magnetic property can be achieved through physical or chemical treatment of the red blood cells. Thus, a sample containing one or more red blood cells and one or more megakaryocytes and/or CTCs can be enriched for the megakaryocytes and/or CTCs by first inducing a magnetic property in the red blood cells and then separating the red blood cells from the megakaryocytes and/or CTCs by flowing the sample through a magnetic field (uniform or non-uniform).

For example, a blood sample can flow first through a size based separation module to remove enucleated cells and cellular components (e.g., analytes having a hydrodynamic size less than 6 $\mu$ms) based on size. Subsequently, the enriched nucleated cells and red blood cells are treated with a reagent, such as $CO_2$, $N_2$, or $NaNO_2$, that changes the magnetic property of the red blood cells' haemoglobin. The treated sample then flows through a magnetic field (e.g., a column coupled to an external magnet), such that the paramagnetic analytes (e.g., red blood cells) will be captured by the magnetic field while the megakaryocytes and/or CTCs and any other non-red blood cells will flow through the device to result in a sample enriched in megakaryocytes and/or CTCs. Additional examples of magnetic separation modules are described in US Application Serial No. 11/323,971, filed December 29, 2005 entitled "Devices and Methods for Magnetic Enrichment of Cells and Other Particles" and US Application Serial No. 11/227,904, filed September 15, 2005, entitled "Devices and Methods for Enrichment and Alteration of Cells and Other Particles".

Subsequent enrichment steps can be used to separate the cells of interest (e.g. megakaryocytes and/or CTCs) from the other cell types such as nucleated red blood cells. A sample enriched by size- and/or deformability-based separation followed by affinity/magnetic separation may be further enriched for cells of interest using fluorescence activated cell sorting (FACS) or selective lysis of a subset of the cells.

When the analyte desired to be separated (e.g., megakaryocytes and/or CTCs) is not ferromagnetic or does not have a potential magnetic property, a magnetic particle (e.g., a bead) or compound (e.g., $Fe^{3+}$) can be coupled to the analyte to give it a magnetic property. In some embodiments, a bead coupled to an antibody that selectively binds to an analyte of interest can be decorated with an antibody elected from the group of anti-CD34, anti-CD41, anti-CK and anti-vimentin. In some embodiments a magnetic compound, such as $Fe^{3+}$, can be couple to an antibody such as those described above. The magnetic particles or magnetic antibodies herein may be coupled to any one or more of the devices herein prior to contact with a sample or may be mixed with the sample prior to delivery of the sample to the device(s). Magnetic particles can also be used to decorate one or more analytes (cells of interest or not of interest) to increase the size prior to performing size- and/or deformability-based separation.

Magnetic fields used to separate analytes/cells in any of the embodiments herein can uniform or non-uniform as well as external or internal to the device. An external magnetic field is one whose source is outside a device herein (e.g., container, channel, obstacles). An internal magnetic field is one whose source is within a device. An example of an internal magnetic field is one where magnetic particles may be attached to obstacles present in the device (or manipulated to create obstacles) to increase surface area for analytes to interact with to increase the likelihood of binding. Analytes captured by a magnetic field can be released by demagnetizing the magnetic regions retaining the magnetic particles. For selective release of analytes from regions, the demagnetization can be limited to selected obstacles or regions. For example, the magnetic field can be designed to be electromagnetic, enabling turn-on and turn-off off the magnetic fields for each individual region or obstacle at will.

In some cases, a fluid sample such as a blood sample is first flowed through one or more size-and/or deformability-based separation modules. Such modules may be fluidly connected in series and/or in parallel. For example a first outlet from a separation module can be fluidly coupled to a capture module. The separation module and capture module may be integrated such that a plurality of obstacles acts both to deflect certain analytes according to size and direct them in a path different than the direction of analyte(s) of interest, and also as a capture module to capture, retain, or bind certain analytes based on size, affinity, magnetism or other physical property.

In some embodiments, the enrichment steps performed have a specificity and/or sensitivity greater than 10, 20, 30, 40, 50, 60, 70, 80, 90, 95, 96, 97, 98, 99, 99.1, 99.2, 99.3, 99.4, 99.5, 99.6, 99.7, 99.8, 99.9 or 99.95% The retention rate of the enrichment module(s) herein is such that $\geq$ 10, 20, 30, 40, 50, 60, 70, 80, 90, 91, 92, 93, 94, 95, 96, 97, 98, 99, or 99.9 % of the analytes or cells of interest (e.g., megakaryocytes and/or CTCs) are retained. Simultaneously, the enrichment modules are configured to remove $\geq$ 10, 20, 30, 40, 50, 60, 70, 80, 85, 90, 91, 92, 93, 94, 95, 96, 97, 98, 99, or 99.9 % of all unwanted analytes (e.g., red blood-platelet enriched cells) from a sample.

For example, the analytes of interest may be retained in an enriched solution that is less than 50, 40, 30, 20, 10, 9.0, 8.0, 7.0, 6.0, 5.0, 4.5, 4.0, 3.5, 3.0, 2.5, 2.0, 1.5, 1.0, or 0.5 fold diluted from the original sample. Any or all of the enrichment steps may increase the concentration of the analyte of interest (megakaryocytes and/or CTCs), for example, by transferring them from the fluid sample to an enriched fluid sample (sometimes in a new fluid medium, such as a buffer).

Methods of detection

In some embodiments, the presence or absence of CD34, CD41, CD45, CK and/or vimentin is determined by flow cytometry, immunofluorescence or immunohistochemistry. The flow cytometry, immunofluorescence or immunohistochemistry may be performed by any suitable method known in the art. In some cases, it is not necessary to isolate or enrich the CTCs and/or the megakaryocytes. The number of cells may simply be counted based on expression of any of the markers described herein. The counting may be automated. The counting may be performed by a computer programme.

In some embodiments, the method further comprises measuring genomic alteration in the megakaryocytes and/or the CTCs. The measuring genomic alteration may confirm the identity of the megakaryocytes and/or the CTCs. For example, the measuring genomic alteration determines that the megakaryocytes are polyploid and/or the measuring genomic alteration may identify one or more mutations of the CTCs and/or confirm the malignancy of the CTCs.

In some embodiments, the measuring genomic alteration comprises use of one or more nucleic acid molecule which hybridises under stringent conditions to a target genomic region. Accordingly, the nucleic acid molecule may be a primer or a probe.

In some embodiments, the nucleic acid molecule detects a genomic alteration selected from the group consisting of polyploidy, a copy number gain, a copy number loss and a rearrangement event. For example, the one or more nucleic acid molecule detects a genomic alteration selected from the group consisting of AR gain, *PTEN* loss, ERG rearrangement, NKX3.1 loss, *C-MYC* gain, *RB1* loss, *CCND1* gain, 6q16 loss and 16q22.1 loss.

Accordingly, in some embodiments the target genomic region is selected from the group consisting of *AR, PTEN, ERG, NXK3.1, C-MYC, RB1, CCND1,* 6q16 and 16q22.1. However, the skilled person will understand that any suitable target genomic region may be selected.

In some embodiments, the methods of the invention may further comprise comparing genomic alteration in the CTCs of the sample from the subject with genomic alteration in the CTCs of a control. The control may be a sample from a healthy individual. The comparison may be made by any suitable means known to the person skilled in the art. The comparison may assist in identifying one or more mutations and/or confirming that the CTCs are malignant.

Subjects

As used herein, the terms "subject" and "patient" are used interchangeably to refer to a human or a non-human mammal. The subject may be a companion non-human mammal (i.e. a pet, such as a dog, a cat, a guinea pig, or a non-human primate, such as a monkey or a chimpanzee), an agricultural farm animal mammal, e.g. an ungulate mammal (such as a horse, a cow, a pig, or a goat) or a laboratory non-human mammal (e.g., a mouse and a rat). The invention may find greatest application in connection with the treatment of male human subjects.

In any of the embodiments herein, the subject may be a human. The subject may be a subject undergoing treatment for prostate cancer.

Prognosis

A "prognosis" is a prediction of the probable course and outcome of a clinical condition or disease. A prognosis of a patient is usually made by evaluating factors, markers, and/or symptoms of a disease that are indicative of a favourable or unfavourable course or outcome of the disease.

In general, a poor prognosis may be characterised as a being the increased risk for a subject of developing an aggressive cancer which, if left untreated, would lead to early death, for example death within 5 years of diagnosis, or death over a 5 to 9 year period from diagnosis. A poor prognosis may therefore mean an increased risk of death as compared to subjects who have a low amount of megakaryocytes and/or a high amount of CTCs in a blood sample. A good prognosis is may therefore represent an assessment that a subject will respond well to therapy with a good chance of medium- to long-term survival over, for example a 5 to 9 or longer year period from diagnosis.

The definition of "poor" or "good" prognosis is dependent on the context, and the skilled person would readily understand these terms. In the context of localised prostate cancer, a poor prognosis may be characterised as a being the increased risk for a subject of developing an aggressive cancer which, if left untreated, would lead to early death, for example death within 5 years or death over a 5 to 9 year period. A poor prognosis may therefore mean an increased risk of death as compared to subjects who have a low amount of megakaryocytes and/or a high amount of CTCs in a blood sample. A good prognosis is may therefore represent an assessment that a subject will have a good chance of medium- to long-term survival, for example over a 5 to 9 or longer year period without any treatment.

In the context of advanced prostate cancer, such as CRPC, a poor prognosis may be characterised as a being the increased risk for a subject of developing an aggressive cancer which, if left untreated, would lead to early death, for example death within a 2 month period or death over a 2 to 16 month period. A poor prognosis may therefore mean an increased risk of death as compared to subjects who have a low amount of megakaryocytes and/or a high amount of CTCs in a blood sample. A good prognosis in the context of advanced prostate cancer such as CRPC may therefore represent an assessment that a subject will respond well to therapy with a good chance of short- to medium-term survival, for example over a 2 to 16 month or longer period.

The data presented in the present application indicates that a period of 3 months, 7 months, 8 months or 13 months may be preferred time points where the different prognosis profiles begin to become evident, but population variations suggest that a range of 2 to 16 months may also be generally useful.

The phrase "determining the prognosis" refers to the process by which the course or outcome of a condition in a patient can be predicted. The term "prognosis" does not refer to the ability to predict the course or outcome of a condition with 100% accuracy. Instead, the term "prognosis" refers to an increased probability that a certain course or outcome will occur; that is, that a course or outcome is more likely to occur in a patient exhibiting a given condition, when compared to those individuals not exhibiting the condition. A prognosis may be expressed as the amount of time a patient can be expected to survive. Alternatively, a prognosis may refer to the likelihood that the disease goes into remission or to the amount of time the disease can be expected to remain in remission. Prognosis can be expressed in various ways; for example prognosis can be expressed as a percent chance that a patient will survive after one month, two months, three months, four months, five months, six months, seven months, eight months, nine months, ten months, eleven months, one year, five years, ten years or the like. Alternatively, prognosis may be expressed as the number of months or years on average that a patient can expect to survive as a result of a condition or disease. The prognosis of a patient may be considered as an expression of relativism, with many factors affecting the ultimate outcome. For example, for patients with certain conditions, prognosis can be appropriately expressed as the likelihood that a condition may be treatable or curable, or the likelihood that a disease will go into remission, whereas for patients with more severe conditions prognosis may be more appropriately expressed as likelihood of survival for a specified period of time. Determining prognosis may be based on the amount of the megakaryocytes and/or CTCs and/or the difference between the amount of CTCs and the amount of megakaryocytes in a sample. Determining prognosis may also be based on the comparison of the amount of the megakaryocytes and/or CTCs and/or the difference between the amount of CTCs and the amount of megakaryocytes with a reference or control. Determining prognosis may also be based on whether a threshold amount of CTCs and/or megakaryocytes has been met and/or exceeded. Determining prognosis may also be based on whether a threshold value for the difference between the amount of CTCs and the amount of megakaryocytes has been met and/or exceeded. Accordingly, the methods of the invention may comprise the step of determining prognosis according to the amount of the megakaryocytes and/or CTCs and/or the difference between the amount of CTCs and the amount of megakaryocytes in a sample; and/or the comparison of the amount of the megakaryocytes and/or CTCs and/or the difference between the amount of CTCs and the amount of megakaryocytes with a reference or control; whether a threshold amount of CTCs and/or megakaryocytes has been met and/or exceeded; and/or whether a threshold value for the difference between the amount of CTCs and the amount of megakaryocytes has been met and/or exceeded.

This method can also be used to determine a patient's suitability for treatment by determining the risk of the patient's cancer advancing. However, if the levels of expression indicate the tumour will remain indolent, it may be decided that particular interventions (such as surgery) are not necessary, and the adverse side-effects of such treatment can be avoided. The methods can also determine the likelihood a patient will relapse.

Cancers

In the present invention, the cancer is prostate cancer. The prostate cancer may be CRPC.

The subject may be undergoing treatment for cancer. The treatment may be any suitable treatment for the cancer affecting the subject, including any treatment described herein. In some embodiments, the cancer has progressed after the subject has undergone treatment for the cancer. A cancer which has progressed may be a cancer which has metastasised, a cancer where one or more tumours has increased in size and/or where the symptoms of the cancer are worsening or becoming less manageable for the patient.

Prostate cancer can be classified according to The American Joint Committee on Cancer (AJCC) tumour-nodes-metastasis (TNM) staging system. The T score describes the size of the main (primary) tumour and whether it has grown outside the prostate and into nearby organs. The N score describes the spread to nearby (regional) lymph nodes. The M score indicates whether the cancer has metastasised (spread) to other organs of the body:

T1 tumours are too small to be seen on scans or felt during examination of the prostate - they may have been discovered by needle biopsy, after finding a raised PSA level. T2 tumours are completely inside the prostate gland and are divided into 3 smaller groups:

T2a - The tumour is in only half of one of the lobes of the prostate gland;
T2b - The tumour is in more than half of one of the lobes;
T2c - The tumour is in both lobes but is still inside the prostate gland.

T3 tumours have broken through the capsule (covering) of the prostate gland- they are divided into 2 smaller groups:

T3a - The tumour has broken through the capsule (covering) of the prostate gland;
T3b - The tumour has spread into the seminal vesicles.

T4 tumours have spread into other body organs nearby, such as the rectum (back passage), bladder, muscles or the sides of the pelvic cavity. Stage T3 and T4 tumours are referred to as locally advanced prostate cancer.

Lymph nodes are described as being 'positive' if they contain cancer cells. If a lymph node has cancer cells inside it, it is usually bigger than normal. The more cancer cells it contains, the bigger it will be:

NX - The lymph nodes cannot be checked;
N0 - There are no cancer cells in lymph nodes close to the prostate;
N1 - There are cancer cells present in lymph nodes.

M staging refers to metastases (cancer spread):

M0 - No cancer has spread outside the pelvis;
M1 - Cancer has spread outside the pelvis;
M1a - There are cancer cells in lymph nodes outside the pelvis;
M1b - There are cancer cells in the bone;
M1c - There are cancer cells in other places.

Prostate cancer can also be scored using the Gleason grading system, which uses a histological analysis to grade the progression of the disease. A grade of 1 to 5 is assigned to the cells under examination, and the two most common grades are added together to provide the overall Gleason score. Grade 1 closely resembles healthy tissue, including closely packed, well-formed glands, whereas grade 5 does not have any (or very few) recognisable glands. Scores of less than 6 or 6 have a good prognosis, whereas scores of more than 6 are classified as more aggressive. The Gleason score was refined in 2005 by the International Society of Urological Pathology and references herein refer to these scoring criteria (Epstein JI, Allsbrook WC Jr, Amin MB, Egevad LL; ISUP Grading Committee. The 2005 International Society of Urological Pathology (ISUP) Consensus Conference on Gleason grading of prostatic carcinoma. Am J Surg Pathol 2005;29(9): 1228-42). The Gleason score is detected in a biopsy, i.e. in the part of the tumour that has been sampled. A Gleason 6 prostate may have small foci of aggressive tumour that have not been sampled by the biopsy and therefore the Gleason is a guide. The lower the Gleason score the smaller the proportion of the patients will have aggressive cancer. Gleason score in a patient with prostate cancer can go down to 2, and up to 10. Because of the small proportion of low Gleasons that have aggressive cancer, the average survival is high, and average survival decreases as Gleason increases due to being reduced by those patients with aggressive cancer (i.e. there is a mixture of survival rates at each Gleason score).

The Gleason Score is the most widespread method of prostate cancer tissue grading used today. It is one determinant of a patient's specific risk of dying due to prostate cancer. Hence, once the diagnosis of prostate cancer is made on a biopsy, tumour grading, especially the Gleason score, is often then relied upon in considering options for therapy. However, the use of the Gleason score is limited by the invasive procedure of acquiring tissue samples which may cause psychological and physical burdens to patients.

The Gleason scoring system is based upon microscopic tumour patterns that are measured by a pathologist, based on a prostate biopsy. Several markers are observed, and then, additional ones are added for a final sum. (The "Gleason Score" and the "Gleason Sum" are same). The Gleason Score is the sum of the primary Gleason grade and the secondary Gleason grades.

When PCa is present in the biopsy, the Gleason score is based upon the degree of loss of the normal glandular tissue architecture (i.e. shape, size and differentiation of the glands) as originally described and developed by Dr. Donald Gleason in 1974 (Gleason DF, and Mellinger GT, J Urol 111:58-64, 1974).

The classic Gleason scoring diagram shows five basic tissue patterns that are technically referred to as tumour "grades". The subjective microscopic determination of this loss of normal glandular structure caused by the cancer is abstractly represented by a grade, a number ranging from 1 to 5, with 5 being the worst grade possible. The biopsy Gleason score is a sum of the primary grade (representing the majority of tumour) and a secondary grade (assigned to the minority of the tumour), and is a number ranging from 2 to 10. The higher the Gleason score, the more aggressive the tumour is likely to act and the worse the patient's prognosis.

> Grade 1: the cancerous tissue will closely resemble the normal tissue
> Grade 2: tissue which still has well advanced structures, such as the glands; though they are also much larger and also the tissues are present amongst them.
> Grade 3: tissue still has the recognizable glands; though, the cells are dimmer
> Grade 4: the tissue has hardly any glands which are identifiable
> Grade 5: there are no identifiable glands in the tissue

The Primary Gleason grade has to be greater than 50% of the total pattern seen (i.e. the pattern of the majority of the cancer observed). The Secondary Gleason grade has to be less than 50%, but at least 5%, of the pattern of the total cancer observed. The sum of the primary and secondary Gleason grades is shown as the Gleason score or sum (i.e. primary grade + secondary grade = GS; i.e. 4+3 or 3+4 = GS 7).

Prostate cancers can also be staged according to how advanced they are. This is based on the TMN scoring as well as any other factors, such as the Gleason score and/or the PSA test. The staging can be defined as follows:

> Stage I:
>
> T1, N0, M0, Gleason score 6 or less, PSA less than 10
> OR
> T2a, N0, M0, Gleason score 6 or less, PSA less than 10
>
> Stage IIA:
>
> T1, N0, M0, Gleason score of 7, PSA less than 20
> OR
> T1, N0, M0, Gleason score of 6 or less, PSA at least 10 but less than 20:
> OR
> T2a or T2b, N0, M0, Gleason score of 7 or less, PSA less than 20
>
> Stage IIB:
>
> T2c, N0, M0, any Gleason score, any PSA
> OR
> T1 or T2, N0, M0, any Gleason score, PSA of 20 or more
> OR
> T1 or T2, N0, M0, Gleason score of 8 or higher, any PSA
>
> Stage III:
> T3, N0, M0, any Gleason score, any PSA
>
> Stage IV:
> T4, N0, M0, any Gleason score, any PSA
> OR
>
> Any T, N1, M0, any Gleason score, any PSA:
> OR
>
> Any T, any N, M1, any Gleason score, any PSA

In the present invention, references herein to "aggressive prostate cancer" can refer to cancers having a Gleason score of more than 6, for example 7 or more or, in some cases, 8 or 9 or more. Aggressive prostate cancer may also be CRPC.

The invention may also be useful for detecting or diagnosing stage II to stage IV cancer or prostate cancer having a Gleason score of 6 or more, 7 or more or 8 or more or CRPC.

It may be determined that a patient has aggressive prostate cancer by histological analysis. Alternatively (or additionally), a PSA level of more than 15, in particular more than 20 ng/ml may be indicative of aggressive prostate cancer, in particular in combination with higher Gleason scores. The level of risk (and hence aggressiveness) may be measured according to the National Institute for Health and Care Excellence guidelines on "Prostate Cancer: diagnosis and treatment" (document CG175 published January 2014), as follows:

Table 1

| Level of risk | PSA | | | Gleason score | | Clinical stage |
|---|---|---|---|---|---|---|
| Low risk | <10 ng/ml | and | | $\leq$6 | and | T1-T2a |
| Intermediate risk | 10-20 ng/ml | or | | 7 | or | T2b |
| High risk[1] | >20 ng/ml | or | | 8-10 | or | $\geq$T2c |
| [1]High-risk localised prostate cancer is also included in the definition of locally advanced prostate cancer. | | | | | | |

The above criteria were first descried by D'Amico et al., JAMA, 1998, 280:969-74. The present invention is useful in providing further information to the stratification shown in Table 1 and can provide a more reliable indication of which prostate cancers will progress and need treatment. The present invention is particularly useful in determining prognosis in patients with an intermediate or high risk of recurrence of disease and/or in determining prognosis in patients with CRPC.

Accordingly, in some embodiments the prostate cancer has been treated by hormone deprivation and the prostate cancer has one or more features selected from the group consisting of increased PSA concentration, size and number of metastases during treatment, a serum testosterone concentration of less than 50 ng/mL, a PSA rise of 25% or more from nadir and consecutive increases in PSA in measurements taken at least three weeks apart.

Measuring the number of metastases may performed in any suitable manner known to the person skilled in the art. For example, measuring the number of metastases involve performing a scan and/or taking a biopsy. The biopsy may be a sample from the subject. The scanning method and/or sample collection site may be selected on the basis of the tissues to which the cancer is known to metastasise. For instance, prostate cancer is known to metastasise to bone, lymph nodes, the urethra, the bladder, the ureters, the rectum, the lungs and the liver. Therefore the scan for detecting prostate cancer metastases may be a scan of bone, lymph nodes, urethra, bladder, ureters, rectum, lungs and/or liver. Accordingly, the sample collection site for detecting prostate cancer metastases may be a bone, lymph nodes, the urethra, the bladder, the ureters, the rectum, the lungs and/or the liver. The most common site of prostate cancer metastasis is bone. Therefore the scan for detecting prostate cancer metastases may be a bone scan. When metastasis to bone is of interest, the scan may be a radionuclide bone scan and computed tomography (CT). The sample may be a bone marrow biopsy.

PSA may be measured accordingly to methods known in the art and/or the methods described herein. PSA measurement is typically by immunoassay. The immunoassay may be a two-site immunoenzymatic "sandwich" assay such as the Access Hybritech PSA assay (Beckman Coulter, Fullerton, CA.) The immunoassay may be a chemoluminscent, chromogenic or florescent assay.

Accordingly, measuring the amount of PSA may comprise use of one or more anti-PSA antibodies. The anti-PSA antibodies may be raised in any suitable animal such as mouse, rabbit, rat, hamster, chicken or goat. The anti-PSA antibodies may be labelled. For example, the anti-PSA antibodies may be conjugated to an enzymatic label such as alkaline phosphatase or to paramagnetic particles.

Measuring the amount of PSA may also comprise use of a substrate. The substrate may be a chemolumiescent substrate, such as Lumi-Phos** 530. Typically, the light production by reaction of the chemolumiescent substrate with the enzymatic label is proportional to the concentration of PSA in the sample. The amount of PSA in the sample may be determined by means of a multipoint calibration curve. Use of a calibration curve may comprise use of a weighted four parameter standard curve with a direct relationship of measured light produced (RLU) to concentration of PSA protein in the sample.

The amount of PSA may be expressed as a concentration. Typically the amount of PSA is expressed as a concentration in ng/mL. The amount of PSA typically refers to the serum concentration of PSA. Accordingly, the methods of the invention may comprise determining a serum PSA concentration. The amount of PSA may alternatively be expressed as, for example, a concentration in whole blood. The concentration in whole blood may be determined by direct measurement of other means. For example, the whole blood concentration of PSA may be proportionally adjusted from the serum concentration of PSA in a manner known to the person skilled in the art.

In some embodiments of the invention, the analysis may be two-fold; first, the presence of prostate cancer is determined, and second the prognosis for the patient is determined to decide what (if any) treatment should be given. The analysis may further comprise monitoring a patient's response to treatment.

Aggressive prostate cancer can be defined as a cancer that requires treatment to prevent, halt or reduce disease progression and potential further complications (such as metastases or metastatic progression). Ultimately, aggressive prostate cancer is prostate cancer that, if left untreated, will kill a patient. Hence the present invention is useful in diagnosing or detecting malignant or metastatic prostate cancer, or determining the prognosis for a patient by identifying patients at risk of malignant or metastatic prostate cancer. In some embodiments of the invention, "aggressive prostate cancer" may be determined by histological analysis. Aggressive prostate cancer may be CRPC.

In some embodiments of the invention, the method of the invention may be combined with another test such as the PSA test or PCA3 test to decrease the possibility of false positive or false negative results. Other tests may be a histological examination to determine the Gleason score, or an assessment of the stage of progression of the cancer.

In some embodiments, the methods of the invention further comprise measuring PSA concentration and/or primary Gleason score in one or more samples from the subject and/or measuring the number of metastases in the subject. Measuring PSA concentration and/or primary Gleason score may be particularly useful when the cancer is prostate cancer.

In some embodiments, the prostate cancer has one or more advanced features selected from the group consisting of increased PSA concentration during treatment, a PSA concentration greater than or equal to 0.2 ng/mL, a primary Gleason score greater than or equal to 7 and greater than or equal to 5 metastases. The prostate cancer with one or more of the advanced features as defined herein may be a biochemical recurrence of prostate cancer after radical prostatectomy.

Following successful surgery (radical prostatectomy), PSA should be undetectable in blood samples (i.e. zero PSA, not 0-4 ng/dl) after about a month. However, some men will have a very low non-rising PSA after surgery, which can sometimes be related to normal prostate tissue left behind. This is uncommon, and referred to as benign regeneration. However, the most widely accepted definition of a cancer recurrence is a PSA > 0.2 ng/mL that has risen on at least two separate occasions at least two weeks apart and measured by the same lab. Accordingly, in some embodiments, increased PSA concentration during treatment comprises a PSA concentration greater than 0.2 ng/mL or has risen on at least two separate occasions at least two weeks apart.

In the post-radiation therapy setting, the most widely accepted definition a cancer recurrence is a PSA concentration that has risen from undetectable in at least three consecutive tests conducted at least two weeks apart and measured by the same lab. Some believe that failure after radiation is not clear until the PSA has risen 2 points above its lowest value after radiation. Accordingly, in some embodiments, increased PSA concentration during treatment comprises a PSA concentration that has risen in at least three consecutive tests conducted at least two weeks apart.

PSA velocity or PSA doubling time, both of which measure the rate at which PSA concentration increases, can be a very significant factor in determining is the aggressiveness of prostate cancer. Men with a shorter PSA doubling time or a more rapid PSA velocity after initial therapy tend to have more aggressive disease, and are therefore more likely to need more aggressive therapies. Likewise, men who have recurrence quickly after surgery (i.e. within 3 years) have a higher risk of aggressive disease.

## Methods of treatment

The present disclosure also provides a method of treating cancer in a subject whose prognosis has been determined according to the invention, further comprising administering a therapeutic agent to the subject and/or adopting a therapeutic regimen.

The therapeutic regimen may be selected from the group consisting of second line hormone therapy, hormone therapy, chemotherapy, radiotherapy, immunotherapy, bone-targeting therapy and induction of megakaryocytes.

As used herein, second line hormone therapy may refer to treatment with a therapeutic agent selected from the group consisting of anti-androgen, such as bicalutamide; anti-androgen withdrawal; corticosteroids such as dexamethasone, prednisolone, or hydrocortisone; triamcinolone; Ketoconazole; transdermal/oral oestrogen, such as Evorel or diethylstilbestrol.

As used herein, hormone therapy may refer to treatment with a therapeutic agent selected from the group consisting of Abiraterone or Enzalutamide.

As used herein, chemotherapy may refer to treatment with a therapeutic agent selected from the group consisting of Docetaxel; Mitoxantrone; Paclitaxel; CL56 (Chlorambucil + lomustine); Estramustine (Estracyt); Melphalan; ECarboF (Epirubicin + Carboplatin + Folinic Acid + 5-Fluorouracil), ECarboX (Epirubicin + Carboplatin + Folinic Acid + Capecitabine) or Cabazitaxel.

As used herein, palliative radiotherapy may refer to radium 223 therapy (a type of internal radiotherapy treatment); local palliative external radiotherapy.

As used herein, immunotherapy may refer to Sipuleucel-T treatment.

As used herein, bone-targeting therapy may refer to drugs used to treat secondary bone cancer, such as Zoledronate, Clodronate, and Denosumab.

Accordingly, the therapeutic agent may be selected from the group consisting of megakaryocytes; an anti-androgen, such as bicalutamide; a corticosteroid such as dexamethasone, prednisolone, or hydrocortisone; triamcinolone; ketoconazole; transdermal or oral oestrogen, such as Evorel or diethylstilbestrol; Abiraterone; Enzalutamide; a chemotherapeutic agent such as Docetaxel, Mitoxantrone, Paclitaxel, CL56 (Chlorambucil + lomustine), Estramustine (Estracyt), Melphalan, ECarboF (Epirubicin + Carboplatin + Folinic Acid + 5-Fluorouracil), ECarboX (Epirubicin + Carboplatin + Folinic Acid + Capecitabine); or Cabazitaxel.

The induction of megakaryocytes may be induction of megakaryocytes *in vivo.* The induction of megakaryocytes *in vivo* may be by administration of a ligand, chemical and/or drug selected from the group consisting of thrombopoietin, GM-CSF, IL-3, IL-6, IL-11, chemokines (SDF-1, FGF-4), LIF, erythropoietin and cyclophosphamide.

The therapeutic regimen and/or therapeutic agent may be selected on the basis of the prognosis of the patient, as determined by the method of the invention. For instance, a different therapeutic regimen and/or therapeutic agent may be selected if the prognosis is poor than if the prognosis is good and/or not poor.

The methods of the invention may optionally comprise a step of comparing the amount of megakaryocytes and/or CTCs and/or the difference between the amount of CTCs and the amount of megakaryocytes with a control or reference.

The present disclosure also provides a method for treatment of cancer comprising:

(a) administering a therapeutic agent and/or adopting a therapeutic regime to a patient,
(b) determining the amount of the megakaryocytes and/or CTCs and/or the difference between the amount of CTCs and the amount of megakaryocytes in a sample from the patient,
(c) comparing the amount of the megakaryocytes and/or CTCs and/or the difference between the amount of CTCs and the amount of megakaryocytes in a sample to a reference or control,
(d) continuing, modifying or discontinuing treatment on the basis of the comparison.

Optionally, the method of treatment of cancer further comprises a step of obtaining a sample from the patient. One or more samples may be obtained. For example, a first sample may be obtained prior to step (a) and a second sample may be obtained after step (a) and prior to step (b).

There is also disclosed herein a method of treating or preventing cancer (for example carcinoma, breast cancer, prostate cancer, lung cancer, pancreatic cancer and/or colon cancer), in a patient, comprising quantifying the amount of megakaryocytes and/or CTCs and/or the difference between the amount of CTCs and the amount of megakaryocytes, in a blood sample obtained from a patient, comparing the values to a reference for each of the cell types, and, if the detected values are greater than the reference, administering alternative treatment for the cancer. Methods of treating cancer may include administering secondary hormone therapy, new type of hormone therapy, chemotherapy and/or radiotherapy to the patient. The amount of megakaryocytes and/or CTCs and/or the difference between the amount of CTCs and the amount of megakaryocytes may be quantified by any suitable means known to the skilled person, for example immunoassay, FISH, flow cytometry, immunofluorescence and/or immunohistochemistry. PSA levels may additionally be quantified in such methods.

The methods of treating cancer of the present disclosure are particularly useful in the treatment of aggressive cancer and/or patients that have a poor prognosis. The methods of treatment may be performed on patients who have been identified as having a particular amount of megakaryocytes and/or CTCs and/or the difference between the amount of CTCs and the amount of megakaryocytes in a blood sample. Said amount is one that it is indicative of aggressive cancer, such as aggressive prostate cancer.

The method of detection etc. may further comprise treating a patient for prostate cancer if prostate cancer is detected or suspected. If prostate cancer is detected or suspected based on the analysis of a blood sample, the presence of prostate cancer can be confirmed by, for example, detecting the presence and/or amount of one or more biomarkers in a sample of prostate tissue. Methods of the disclosure may therefore further comprise a step of detecting or determining the amount of a biomarker in a prostate tissue sample. The prostate tissue sample may have been obtained previously from a patient, or the method may comprise a step of obtaining or providing said prostate tissue sample. Analysis of prostate tissue samples may also comprise a histological analysis, and any of the methods herein may be combined with a histological analysis to assist in the diagnosis.

In some cases treatment for cancer involves resecting the tumour or other surgical techniques. For example, treatment for prostate cancer may comprise a radical prostatectomy, or bilateral orchiectomy. Treatment may alternatively or additionally involve treatment by chemotherapy and/or radiotherapy. Chemotherapeutic treatments include docetaxel and estramustine. Radiotherapeutic treatments include external beam radiotherapy, brachytherapy, or, as the case may be, prophylactic radiotherapy. Other treatments include abiraterone, enzalutamide, prednisolone, hormone therapy (including gonadorelin analogues such as buserelin, goserelin, histrelin, leuproelin and triptorelin and also including gonadotrophin-releasing hormone antagonists such as degarelix), anti-androgen treatment (such as androgen deprivation therapy using for example cyproterone acetate, flutamide, bicalutamide and abiraterone acetate), cryotherapy, high-intensity focused ultrasound, and/or administration of bisphosphonates and/or steroids. Palliative therapies include irradiation and strontium.

Additional methods

The inventors foresee a number of further applications, including those disclosed below.

There is disclosed herein a method for determining the suitability of a patient for treatment for cancer (for example carcinoma, breast cancer, prostate cancer, lung cancer, pancreatic cancer and/or colon cancer), comprising detecting the amount of the megakaryocytes and/or CTCs and/or the difference between the amount of CTCs and the amount of megakaryocytes in a blood sample, optionally comparing the amount of cells with a control, and deciding whether or not to proceed with treatment for cancer is diagnosed or suspected, in particular if aggressive cancer is diagnosed or suspected.

There is also disclosed a method of monitoring a patient's response to therapy, comprising determining the amount of the megakaryocytes and/or CTCs and/or the difference between the amount of CTCs and the amount of megakaryocytes in a blood sample, obtained from a patient that has previously received therapy for a cancer (for example chemotherapy and/or radiotherapy). The amount of the megakaryocytes and/or CTCs and/or the difference between the amount of CTCs and the amount of megakaryocytes may be compared with the amount of megakaryocytes and/or CTCs and/or the difference between the amount of CTCs and the amount of megakaryocytes in a sample obtained from a patient before receiving the therapy for cancer. A decision can then be made on whether to continue the therapy or to try an alternative therapy based on the comparison of the megakaryocytes and/or CTCs and/or the difference between the amount of CTCs and the amount of megakaryocytes.

Accordingly, the disclosure also provides a method for monitoring a patient's response to therapy comprising:

(a) determining the amount of the megakaryocytes and/or CTCs and/or the difference between the amount of CTCs and the amount of megakaryocytes in a first sample obtained from a patient prior to administration of the therapy; and
(b) determining the amount of the megakaryocytes and/or CTCs and/or the difference between the amount of CTCs and the amount of megakaryocytes in a second sample obtained from a patient after administration of the therapy.

The method may optionally further comprise a step of administering the therapy (for example administering a therapeutic agent and/or adopting a therapeutic regime). The step of administering the therapy may be performed between steps (a) and (b) above.

There is also disclosed herein a method comprising:

a) determining the amount of the megakaryocytes and/or CTCs and/or the difference between the amount of CTCs and the amount of megakaryocytes in a blood sample, obtained from a patient that has previously received therapy for a cancer;
b) comparing the amount of the megakaryocytes and/or CTCs and/or the difference between the amount of CTCs and the amount of megakaryocytes determining in step a) with a previously determined amount of the megakaryocytes and/or CTCs and/or the difference between the amount of CTCs and the amount of megakaryocytes ; and
c) maintaining, changing or withdrawing the therapy for cancer.

The methods may comprise a prior step of administering the therapy for cancer to the patient. The method may also comprise a pre-step of determining the amount of the megakaryocytes and/or CTCs and/or the difference between the amount of CTCs and the amount of megakaryocytes in a blood sample obtained from the same patient prior to administration of the therapy. In step c), the therapy for cancer may be maintained if an appropriate adjustment in the amount of the megakaryocytes and/or CTCs and/or the difference between the amount of CTCs and the amount of megakaryocytes is determined. For example, if there is an increase in the amount of megakaryocytes and/or a reduction in the amount of CTCs and/or a reduction in the difference between the amount of CTCs and the amount of megakaryocytes, then treatment may be maintained. Alternatively, treatment may not need to be changed. If the amount of the megakaryocytes and/or CTCs and/or the difference between the amount of CTCs and the amount of megakaryocytes have altered sufficiently, for example back to what may be considered healthy or low-risk levels, then treatment for cancer may be withdrawn. Alternatively, it may be necessary to continue until therapy for a number of months or half a year until any signs of cancer disappear. If the amount of the megakaryocytes and/or CTCs and/or the difference between the amount of CTCs and the amount of megakaryocytes are unchanged or have worsened (for example there is an increase in the amount of CTCs and/or a decrease in the amount of megakaryocytes and/or a an increase in the difference between the amount of CTCs and the amount of megakaryocytes, this may be indicative of a worsening of the patient's condition, and hence an alternative therapy for cancer may be attempted. In this way, drug candidates useful in the treatment of cancer (in particular aggressive cancer, such as aggressive prostate cancer and/or CRPC) can be screened.

The methods may also comprise a step of determining the risk and/or the rate of cancer progression. The methods may also comprise a step of administering a therapeutic and/or adopting a therapeutic regimen on the basis of the determination of the risk and/or the rate of cancer progression.

The methods may be useful for individualising patient treatment, since the effect of different treatments can be easily monitored, for example by measuring megakaryocytes and/or CTCs and/or the difference between the amount of CTCs and the amount of megakaryocytes in successive blood samples following treatment. The methods of the disclosure can also be used to predict the effectiveness of treatments, such as responses to hormone ablation therapy.

There is also disclosed herein a method identifying a drug useful for the treatment of cancer (for example carcinoma, breast cancer, prostate cancer, lung cancer, pancreatic cancer and/or colon cancer), comprising:

(a) quantifying the amount of the megakaryocytes and/or CTCs and/or the difference between the amount of CTCs and the amount of megakaryocytes in a blood sample obtained from a patient;
(b) administering a candidate drug to the patient;
(c) quantifying the amount of the megakaryocytes and/or CTCs and/or the difference between the amount of CTCs and the amount of megakaryocytes in a blood sample obtained from the same patient at a point in time after administration of the candidate drug; and
(d) comparing the value determined in step (a) with the value determined in step (c), wherein an appropriate change in the amount of the megakaryocytes and/or CTCs and/or the difference between the amount of CTCs and the amount of megakaryocytes between the two samples identifies the drug candidate as a possible treatment for cancer. For example, an increase in the amount of megakaryocytes, or a decrease in the amount of CTCs, or an decrease in the difference between the amount of CTCs and the amount of megakaryocytes may be indicative of the usefulness of the drug candidate in treatment of cancer. The drug may be a compound, an antibody or antibody fragment.

Examples of antibodies are the immunoglobulin isotypes (e.g., IgG, IgE, IgM, IgD and IgA) and their isotypic subclasses; fragments which comprise an antigen binding domain such as Fab, F(ab')$_2$, Fv, scFv, dAb, Fd; and diabodies. Antibodies may be polyclonal or monoclonal. A monoclonal antibody may be referred to herein as "mAb".

Isolated mixtures

The disclosure also provides an isolated mixture of megakaryocytes and CTCs.

As used herein, "isolated" when used in reference to megakaryocytes and CTCs means that a naturally occurring cell has been removed from its normal physiological environment.

As used herein, "mixture" when used in reference to megakaryocytes and CTCs means that any composition comprising megakaryocytes and CTCs. For example, the mixture may be a blood sample comprising megakaryocytes and CTCs, a buffer solution comprising megakaryocytes and CTCs, a cell culture comprising megakaryocytes and CTCs, culture medium comprising megakaryocytes and CTCs and/or an enrichment apparatus or an element thereof comprising megakaryocytes and CTCs. The megakaryocytes and CTCs in the mixture may be alive, dead, fixed, attached to a substrate and/or in suspension.

The isolated mixture may be in any suitable container known to the person skilled in the art. For example, the isolated mixture may be in a culture plate or dish, a vial or a tube.

The isolated mixture may be a cell bank. The cell bank may be maintained by any suitable means known in the art. For example, the isolated mixture and/or cell bank may be in tissue culture or an incubator. Alternatively, the isolated mixture and/or cell bank may be a frozen stock. The isolated mixture and/or cell bank may therefore be stored in a freezer.

The isolated mixture may be derived from a single patient. The isolated mixture may be marked with a patient specific label. The label may serve to identify the isolated sample.

The isolated mixture may be enriched for megakaryocytes and/or CTCs. The enrichment may be performed as described herein.

The isolated mixture may be the result of, or an intermediate of, the process of enriching a sample for megakaryocytes and/or CTCs.

In some embodiments, the megakaryocytes and/or CTCs in the mixture are fixed. Accordingly, the isolated mixture may also be on a cover slip.

In some embodiments, the megakaryocytes and/or CTCs in the mixture are labelled. In some embodiments, the megakaryocytes and/or CTCs are labelled at one or more markers selected from the group consisting of CD34, CD41, CD45, CK and vimentin. For example, the megakaryocytes and/or CTCs may be labelled by the binding of antibodies, antibody fragments and/or aptamers selected from the group consisting of anti-CD34, anti-CD41, anti-CD45, anti-CK and anti-vimentin. The megakaryocytes and/or CTCs in the mixture may also be labelled by a nucleic acid, such as a probe or a primer. Accordingly, the megakaryocytes and/or CTCs in the mixture may be labelled by *in situ* hybridisation such as FISH.

In some embodiments, the FISH is repeated FISH. Repeated FISH is a procedure which involves repeatedly probing a single sample (for example a slide with fixed cells) according to an *in situ* hybridisation interspersed with a stripping step. Stripping may be as defined herein. FISH may be performed as described herein or using a known protocol. Repeated FISH generates genomic alteration information for multiple genomic regions in single cells. Repeated FISH can be used to confirm the malignancy of suspicious CTCs, uncovering the heterogeneity of cancer cells by analysing the differences in genomic alterations between individual cells and correlating genomic alterations with cellular features and different types of CTCs to understand mechanisms of metastases. Accordingly, the FISH may be on the same cells after immunofluorescence analysis. *in situ* hybridisation such as FISH and repeated FISH may be performed on cells after immunofluorescence analysis and stripping. The immunofluorescence analysis may, for example, be used to identify the cells as CTCs or megakaryocytes.

In some embodiments, the FISH or repeated FISH comprises stripping at 40 to 90 °C with a stripping buffer comprising 1 % to 3 % SDS, 0.055 M to 0.075 M Tris-HCI and 0.5 % to 1.1 % β-mercaptoethanol and wherein the stripping buffer has a pH from 6.6 to 7.0. The stripping buffer may comprise 2 % SDS, 0.0625 M Tris-HCI and 0.8 % β-mercaptoethanol, wherein the stripping buffer has a pH of 6.8.

The disclosure provides an isolated mixture of megakaryocytes and/or CTCs for use in the diagnosis of cancer.

The disclosure also provides megakaryocytes for use in the diagnosis of cancer. Each step of the diagnosis of cancer may be performed by any suitable means including the methods described herein.

In some embodiments, the method for the diagnosis of prostate cancer comprises:

(a) measuring the amount of megakaryocytes in a sample from a subject,
(b) comparing the amount of megakaryocytes in the sample to a control or reference,
(c) determining the significance of any difference between the amount of megakaryocytes in the sample and the control or reference,
(d) diagnosing prostate cancer if the amount of megakaryocytes in the sample is significantly higher than the control or reference.

The method of diagnosis may optionally further comprise comparing the CTCs and/or the difference between the amount of CTCs and the amount of megakaryocytes with a control or reference.

Determination of significance may be calculated by any suitable test known to the person skilled in the art, for example a t-test.

In some embodiments, the invention provides a method for the diagnosis of advanced prostate cancer comprising:

(a) measuring the amount of megakaryocytes in a sample from a subject.
(b) comparing the amount of megakaryocytes in the sample to a control or reference,
(c) determining the significance of any difference between the amount of megakaryocytes in the sample and the control or reference,
(d) diagnosing advanced prostate cancer if the amount of megakaryocytes in the sample is significantly higher than the control or reference.

A cancer with a poor prognosis may alternatively be considered to be advanced cancer. Accordingly, any of the methods described herein as methods of determining prognosis of cancer may be used as a method of diagnosing advanced cancer when a poor prognosis is determined.

The methods of determining prognosis, diagnosis and treatment of prostate cancer may further comprise detecting megakaryocytes in a sample, optionally by immunoassay, flow cytometry, immunofluorescence, immunohistochemistry and/or FISH.

The immunoassay, flow cytometry, immunofluorescence, immunohistochemistry and/or FISH may be performed by any suitable method and according to any method described herein. The specific binding molecules described herein may be used in the flow cytometry, immunofluorescence, immunohistochemistry and/or FISH. The megakaryocytes may be detected on the basis of any feature of megakaryocytes defined herein, for example, the megakaryocytes may be detected as cells which are CD34 positive, CD41 positive, CD45 negative, CK negative and vimentin negative, have a nucleus with a dimension that is greater than or equal to 10 μm and are polyploid.

Uses of megakaryocytes

The present disclosure further provides megakaryocytes for use in the treatment of cancer. The megakaryocytes may be autologous. Alternatively, the megakaryocytes may be allogeneic or xenogeneic.

The disclosure also provides the use of circulating megakaryocytes isolated from a subject in an ex *vivo* and/or *in vitro* method of diagnosing or determining prognosis of cancer. The method may also comprise use of CTCs isolated from the subject.

The megakaryocytes may be prepared by any suitable method. There are several approaches to produce megakaryocytes both *in vitro* and *in vivo. In vitro* cell culture methods may be used to generate sufficient megakaryocytes to be transplanted and/or injected into cancer patients.

Megakaryocyte cell lines can be obtained by treating human megakaryocyte lineage leukemia cell lines, such as CMK, Meg01 and K562. K562. These hematopoietic precursor cells may be cultured in Iscove's Modified Dulbecco's Media (Sigma Aldrich) + 10% fetal bovine serum (Gibco, Life technologies) and induced with 50 nM of phorbol myristate acetate (PMA) (Sigma Aldrich) or 12-0-tetradecanoylphorbol-13-acetate to differentiate into mature megakaryocytes.

Megakaryocytes can also be produced by stimulating normal hematopoietic precursor cells with thrombopoietin, GM-CSF, IL-3, IL-6, IL-11, chemokines (SDF-1, FGF-4), LIF, and erythropoietin.

Megakaryocytes with long-term self-renewal capacity can also be established from human embryonic stem cells or human induced pluripotent stem cells derived from human skin fibroblasts, blood cells or other type of cells by controlled expression of certain genes, such as c-MYC, BMI1, and BCL-XL, by gene transfection. These megakaryocyte cell lines may be prepared as described in Nakamura et al., (2014) Cell Stem Cell 14(4), 535 - 548 and are candidate cell banks.

The disclosure further provides a method of treating cancer in a subject comprising administration of megakaryocytes. The method of treatment may be a method of treating a subject in need thereof. The megakaryocytes may be autologous. Alternatively, the megakaryocytes may be allogeneic or xenogeneic. The megakaryocytes may also be induced *in vivo* by administration of a ligand, chemical and/or drug selected from the group consisting of thrombopoietin, GM-CSF, IL-3, IL-6, IL-11, chemokines (SDF-1, FGF-4), LIF, erythropoietin and cyclophosphamide.

The method of treatment can be of a human or an animal subject and the present disclosure extends equally to uses in both human and/or veterinary medicine. The megakaryocytes and/or pharmaceutical composition is preferably administered to an individual in a "therapeutically effective amount", this being sufficient to show benefit to the individual and/or to ameliorate, eliminate or prevent one or more symptoms of cancer. As used herein, "treatment" includes any regime that can benefit a human or non-human animal, preferably a mammal. The treatment may be in respect of an existing condition or may be prophylactic (preventative treatment).

Pharmaceutical compositions

As disclosed herein, megakaryocytes for use in treatment of cancer and/or in a method of treating cancer may be formulated as a pharmaceutical composition comprising megakaryocytes.

The pharmaceutical composition can be formulated for use by any convenient route. The pharmaceutical composition for use in accordance with this aspect of the disclosure will normally include a pharmaceutically acceptable carrier, excipient, diluent, adjuvant, vehicle, buffer or stabiliser in addition to megakaryocytes as defined herein. Such carriers include, but are not limited to, saline, buffered saline such as phosphate buffered saline (PBS), dextrose, liposomes, water, glycerol, polyethylene glycol, ethanol and combinations thereof. This pharmaceutical composition may be in any suitable form depending upon the desired method of administering it to a patient.

The pharmaceutical composition can be provided in unit dosage form, will generally be provided in a sealed container and may be provided as part of a kit. Such a kit would normally (although not necessarily) include instructions for use. It can include a plurality of said unit dosage forms.

The megakaryocytes may be locally administered to the site of the cancer. Accordingly, the pharmaceutical composition can be adapted for administration by any appropriate route, for example by the oral (including buccal or sublingual), rectal, nasal, topical (including buccal, sublingual or transdermal), vaginal or parenteral (including subcutaneous, intramuscular, intravenous, intraperitoneal or intradermal) route, although it will typically be adapted for intravenous administration or direct administration to the site of the cancer. Such compositions can be prepared by any method known in the art of pharmacy, for example by admixing the active ingredient with the carrier(s) or excipient(s) under sterile conditions.

Pharmaceutical compositions adapted for parenteral administration include aqueous and non-aqueous sterile injection solution which can contain anti-oxidants, buffers, bacteriostats and solutes which render the formulation substantially isotonic with the blood of the intended recipient; and aqueous and non-aqueous sterile suspensions which can include suspending agents and thickening agents. Excipients which can be used for injectable solutions include water, alcohols, polyols, glycerine and vegetable oils, for example. The compositions can be presented in unit-dose or multi-dose containers, for example sealed ampoules and vials, and can be stored in a frozen condition (for example as a cell bank) requiring only the addition of the sterile liquid carried, for example water for injections, immediately prior to use.

The pharmaceutical compositions can contain preserving agents, solubilising agents, stabilising agents, wetting agents, emulsifiers, sweeteners, colourants, odourants, salts, buffers, coating agents or antioxidants.

The pharmaceutical compositions for use in the disclosure can also contain one or more other therapeutically active agents in addition to megakaryocytes. The pharmaceutical compositions for use in the disclosure may contain one or more anti-inflammatory or immunomodulatory drugs in addition to the megakaryocytes. Anti-inflammatory or immunomodulatory drugs include (i) steroids such as glucocorticoids, for example prednisone, prednisolone, methylprednisolone, cortisone, hydrocortisone, betamethasone, dexamethasone and triamcinolone, (ii) non-steroidal anti-inflammatory drugs (NSAIDs) such as aspirin, ibuprofen, celecoxib and naproxen, and (iii) anti-inflammatory peptides such as Immune Selective Anti-Inflammatory Derivatives (ImSAIDs).

Dosages

As disclosed herein, megakaryocytes and/or pharmaceutical compositions comprising megakaryocytes may be administered by any suitable means to any suitable location at any suitable dose and/or dosage regime.

Dosages of the megakaryocytes and/or pharmaceutical composition for use in the present disclosure can vary between wide limits, depending upon the disease or disorder to be treated, the age and condition of the individual to be treated, etc. and a physician will ultimately determine appropriate dosages to be used.

This dosage can be repeated as often as appropriate. If side effects develop the amount and/or frequency of the dosage can be reduced, in accordance with normal clinical practice.

For administration to mammals, and particularly humans, it is expected that the daily dosage of the active agent will be 4 or more megakaryocytes per 7.5 mL of the total blood of the subject. Accordingly, if a typical adult subject has a total blood volume of approximately 5 L, the expected daily dosage may be 2667 or more megakaryocytes per patient per day. Alternatively, the daily does may be a dose sufficient to maintain a level of at least 4 or more megakaryocytes per 7.5 mL of the subject's blood. The physician in any event will determine the actual dosage which will be most suitable for an individual which will be dependent on factors including the age, weight, sex and response of the individual. The above dosages are exemplary of the average case. There can, of course, be instances where higher or lower dosages are merited, and such are within the scope of this disclosure.

The megakaryocytes and/or the pharmaceutical composition comprising megakaryocytes may be administered intravenously. The megakaryocytes may be suspended in a physiologically acceptable buffer. A physiologically acceptable buffer is any buffer suitable for administration to a subject. The physiologically acceptable buffer may be an injectable solution such as saline, buffered saline such as phosphate buffered saline (PBS), dextrose, liposomes, water, glycerol, polyethylene glycol, ethanol, artificial blood, donor blood, autologous blood and combinations thereof.

As an alternative to administration of megakaryocytes, the megakaryocytes may be induced *in vivo.* Accordingly, administration of megakaryocytes may not be necessary. Megakaryocytes can be induced by administration of suitable ligands, chemicals and/or drugs selected from the group consisting of thrombopoietin, GM-CSF, IL-3, IL-6, IL-11, chemokines (SDF-1, FGF-4), LIF, erythropoietin and cyclophosphamide.

An example protocol for *in vivo* induction of megakaryocytes may involve administration of thrombopoietin, GM-CSF, IL-3, IL-6, IL-11, chemokines (SDF-1, FGF-4), LIF, erythropoietin and cyclophosphamide to a subject by any suitable route. For example, cyclophosphamide is typically administered orally in tablet form. Alternatively, some factors may be administered by injection. Factors which act as signals for megakaryocyte production, including thrombopoietin, GM-CSF, IL-3, IL-6, IL-11, chemokines (SDF-1, FGF-4), LIF, and erythropoietin, can induce differentiation of progenitor cells in the bone marrow towards a final megakaryocyte phenotype. Therefore they can be used (injected) for *in vivo* induction of megakaryocyte production. Cyclophosphamide may also induce peripheral blood megakaryocyte progenitors.

Stripping buffer

The invention may use a stripping buffer comprising 1 % to 3 % SDS, 0.055 M to 0.075 M Tris-HCl and 0.5 % to 1.1 % β-mercaptoethanol wherein the stripping buffer has a pH from 6.6 to 7.0. In some embodiments, the stripping buffer comprises 2 % SDS, 0.0625 M Tris-HCl and 0.8 % β-mercaptoethanol wherein the stripping buffer has a pH of 6.8. The stripping buffer may for instance be useful in FISH following flow cytometry, immunofluorescence and/or immunohistochemistry protocols.

The stripping buffer may be for use in the diagnosis of cancer.

As used herein, "stripping" describes the removal of specific binding molecules such as primary and/or secondary antibodies. Accordingly, a stripping buffer is a composition that serves the function of removing specific binding molecules such as primary and/or secondary antibodies.

Kits

The disclosure also provides a kit comprising specific binding molecules which bind to one or more of CD34, CD41, CD45, CK and/or vimentin. The specific binding molecules may be antibodies, antibody fragments or aptamers. Accordingly, the kit may comprise anti-CD34, anti-CD41, anti-CD45, anti-CK and/or anti-vimentin antibodies, antibody fragments or aptamers.

The kit may further comprise one or more nucleic acid molecules which hybridises under stringent conditions to a target genomic region. The target genomic region may be selected from the group consisting *of AR, PTEN, ERG, NXK3.1, C-MYC, RB1, CCND1,* 6q16 and 16q22.1. However, the skilled person will understand that any suitable target genomic region may be selected.

The kit may further comprise a stripping buffer comprising 1 % to 3 % SDS, 0.055 M to 0.075 M Tris-HCl and 0.5 % to 1.1 % β-mercaptoethanol, wherein the stripping buffer has a pH from 6.6 to 7.0. The stripping buffer may comprise 2 % SDS, 0.0625 M Tris-HCl and 0.8 % β-mercaptoethanol wherein the stripping buffer has a pH of 6.8.

The kit may further comprise a nuclear dye. Any suitable nuclear dye known to the person skilled in the art may be used, such as DAPI, propidium iodide or a Hoescht nuclear dye such as Hoescht 3342.

Alternatively, , the kit may comprise one or more nucleic acid molecule which hybridises under stringent conditions to a target genomic region, as defined above. Alternatively, the kit may comprise a stripping buffer, as defined above. Alternatively, the kit may comprise a nuclear dye, as defined above.

Any of the kit components defined above may be combined in any combination. For example, the kit may comprise:

- One or more specific binding molecules, as defined herein,
- A stripping buffer, as defined herein,
- One or more nucleic acid molecules, as defined herein,
- A nuclear dye, as defined herein,
- One or more specific binding molecules and a stripping buffer,
- One or more specific binding molecules and one or more nucleic acid molecules,
- One or more specific binding molecules and a nuclear dye,
- A stripping buffer and one or more nucleic acid molecules,
- A stripping buffer and a nuclear dye,
- One or more nucleic acid molecules and a nuclear dye,
- One or more specific binding molecules, a stripping buffer and one or more nucleic acid molecules,
- One or more specific binding molecules, a stripping buffer and a nuclear dye,
- One or more specific binding molecules, one or more nucleic acid molecules and a nuclear dye,
- A stripping buffer, one or more nucleic acid molecules and a nuclear dye, or
- One or more specific binding molecules, a stripping buffer, one or more nucleic acid molecules and a nuclear dye.

For example, the kit may comprise:

- An anti-CD34 specific binding molecule,
- an anti-CD41 specific binding molecule,
- an anti-CK specific binding molecule,
- an anti-vimentin specific binding molecule and optionally,
- a nuclear dye, such as DAPI.

As used herein, a "kit" is a packaged combination optionally including instructions for use of the combination and/or other reactions and components for such use.

The specific binding molecule used in the invention can be a fragment of an antibody. It has been shown that fragments of a whole antibody can perform the function of binding antigens. Examples of binding fragments are (i) the Fab fragment consisting of $V_L$, $V_H$, $C_L$ and $C_H 1$ domains; (ii) the Fd fragment consisting of the $V_H$ and $C_H 1$ domains; (iii) the Fv fragment consisting of the $V_L$ and $V_H$ domains of a single antibody; (iv) the dAb fragment (Ward, E.S. et al., Nature 341:544-546 (1989)) which consists of a $V_H$ domain; (v) isolated CDR regions; (vi) F(ab')$_2$ fragments, a bivalent fragment comprising two linked Fab fragments; (vii) single chain Fv molecules (scFv), wherein a $V_H$ domain and a $V_L$ domain are linked by a peptide linker which allows the two domains to associate to form an antigen binding site (Bird et al., Science 242:423-426 (1988); Huston et al., PNAS USA 85:5879-5883 (1988)); (viii) bispecific single chain Fv dimers (PCT/US92/09965) and (ix) "diabodies", multivalent or multispecific fragments constructed by gene fusion (WO94/13804; P. Hollinger et al., Proc. Natl. Acad. Sci. USA 90: 6444-6448 (1993)). Typically, the fragment is a Fab, F(ab')$_2$ or Fv fragment or an scFv molecule.

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associated with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Hollinger & Winter, Current Opinion Biotechnol. 4:446-449 (1993)), e.g. prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single chain "Janusins" described in Traunecker et al., EMBO Journal 10:3655-3659 (1991).

Bispecific diabodies, as opposed to bispecific whole antibodies, may also be useful because they can be readily constructed and expressed in *E. coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

General definitions

As used herein the terms "measure" and "measuring" are used interchangeably with the terms "count" and "counting" in the context of measuring an amount or number of cells (such as megakaryocytes and/or CTCs). The measuring may be manual (for example visually counting) or automated (for example detecting using a computer system and/or computer implemented method). The measuring may also be semi-automated (for example using a computer system and/or computer implemented method which requires essential inputs from a user or decisions made by a user).

As used herein the term "number" may be used as an alternative to the term "amount". Accordingly, the methods of the invention may comprise a step of measuring the number of megakaryocytes and/or CTCs and/or the difference between the number of CTCs and the number of megakaryocytes in a blood sample.

"About" and "approximately" shall generally mean an acceptable degree of error for the quantity measured given the nature or precision of the measurements. Typically, exemplary degrees of error are within 20 percent (%), preferably within 10%, and more preferably within 5% of a given value or range of values.

Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

As used herein, the term "mutation" may refer to genomic copy number changes and/or changes in genomic structure.

As used herein, "nucleic acid(s)" or "nucleic acid molecule" generally refers to any ribonucleic acid or deoxyribonucleic acid, which may be unmodified or modified DNA or RNA. "Nucleic acids" include, without limitation, single- and double-stranded nucleic acids. As used herein, the term "nucleic acid(s)" also includes DNA as described above that contain one or more modified bases. Thus, DNA with backbones modified for stability or for other reasons are "nucleic acids". The term "nucleic acid(s)" as it is used herein embraces such chemically, enzymatically or metabolically modified forms of nucleic acids, as well as the chemical forms of DNA characteristic of viruses and cells, including for example, simple and complex cells.

As used herein, the terms "hybridizing to" and "hybridization" are interchangeably used with the term "specific for" and refer to the sequence-specific non-covalent binding interactions with a complementary nucleic acid, for example, interactions between a target nucleic acid sequence and a target specific nucleic acid primer or probe. In a preferred embodiment a nucleic acid, which hybridizes, is one which hybridizes with a selectivity of greater than 70 %, greater than 80 %, greater than 90 % and most preferably of 100 % (i.e. cross hybridization with other DNA species preferably occurs at less than 30 %, less than 20 %, less than 10 %). As would be understood to a person skilled in the art, a nucleic acid, which "hybridizes" to the DNA product of a genomic region of the invention, can be determined taking into account the length and composition.

As used herein, "stringent conditions for hybridization" are known to those skilled in the art and can be found in Current Protocols in Molecular Biology, John Wiley & Sons, N.Y., 6.3.1-6.3.6, 1991. Stringent conditions may be defined as equivalent to hybridization in 6X sodium chloride/sodium citrate (SSC) at 45 °C, followed by a wash in 0.2 X SSC, 0.1 % SDS at 65 °C. Alternatively, stringent conditions may be defined as equivalent to hybridization in 50 % v/v formamide, 10 % w/v Dextran sulphate, 2X SSC at 37 °C, followed by a wash in 50 % formamide / 2x SSC at 42 °C.

The term "primer" as used herein refers to a nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e. in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides, although it may contain fewer nucleotides.

As used herein, the term "probe" means nucleic acid and analogues thereof and refers to a range of chemical species that recognise polynucleotide target sequences through hydrogen bonding interactions with the nucleotide bases of the target sequences. The probe or the target sequences may be single- or double-stranded DNA. A probe is at least 8 nucleotides in length and less than the length of a complete polynucleotide target sequence. A probe may be 10, 20, 30, 50, 75, 100, 150, 200, 250, 400, 500 and up to 10,000 nucleotides in length. Probes can include nucleic acids modified so as to have one or more tags which are detectable by fluorescence, chemiluminescence and the like ("labelled probe"). The labelled probe can also be modified so as to have both one or more detectable tags and one or more quencher molecules, for example Taqman® and Molecular Beacon® probes. The nucleic acid and analogues thereof may be DNA, or analogues of DNA, commonly referred to as antisense oligomers or antisense nucleic acid. Such DNA analogues comprise but are not limited to 2-'O-alkyl sugar modifications, methylphosphonate, phosphorothiate, phosphorodithioate, formacetal, 3'-thioformacetal, sulfone, sulfamate, and nitroxide backbone modifications, and analogues wherein the base moieties have been modified. In addition, analogues of oligomers may be polymers in which the sugar moiety has been modified or replaced by another suitable moiety, resulting in polymers which include, but are not limited to, morpholino analogues and peptide nucleic acid (PNA) analogues (Egholm, et al., Peptide Nucleic Acids (PNA)-Oligonucleotide Analogues with an Achiral Peptide Backbone, (1992)).

Preferred features for the second and subsequent aspects of the invention are as for the first aspect of the invention *mutatis mutandis.*

The present invention will now be described by way of reference to the following Examples and accompanying Drawings which are present for the purposes of illustration only and are not to be construed as being limiting on the invention.

Example 1 - Workflow of blood sample process for CTC & megakaryocytes analysis

**Sample collection and preparation:**

A 7.5 mL of whole blood sample are drawn into EDTA Vacutainer tubes (Becton Dickinson and Company, Plymouth, UK) and acquired after getting discarding the initial 2-3 mL of blood to avoid contamination with epithelial cells from the skin. The blood samples are processed within 2 h after collection. Alternatively, they can be stored at room temperature or 4 °C and processed within 24 h of blood draw. If stored at 4 °C, the blood samples are warmed at 25-30 °C for 10-15 min before processing.

**Sample isolation:**

CTCs and megakaryocytes were isolated from peripheral blood mononuclear cells (PBMC) using Parsortix™ system. Details of the steps were shown below.

Whole blood Sample pre-processing, enriching mononuclear cell fraction

1. Fill 50 mL LeucoSep tube with 15.3 mL of Ficoll-Paque PLUS (GE Healthcare), and spin at 1,000g for 30 seconds at room temperature (RT).
2. Prepare 10 mL Ca$^{++}$ and Mg$^{++}$ free PBS (Sigma) to 50 mL conical tube for each 7.5 mL sample. Wet the pipette using PBS and add 1 mL of PBS to the LeucoSep tube to cover the frit.
3. Leave 0.3-0.5 mL of whole blood in EDTA vacutainer and gently transfer 7 mL of whole blood to the LeucoSep tube along the tube wall. Add 8.0 mL of PBS to the tube (to dilute sample and rinse remaining blood in the pipette and blood on the tube wall).
4. Centrifuge 15 min for fresh sample or 20 min for samples stored overnight at 1,000 g. Switch off brakes of centrifuge.
5. After spin, a layer of white cells can be seen just above the frit. Decant the supernatant containing cells from LeucoSep tube to a new 50 mL conical tube.
6. Rinse the same side wall of LeucoSep tube with 5 mL of PBS using 10mL pipette and add to the same 50 mL tube. Be careful not to suction the Ficoll-Paque PLUS through the frit; avoid pressing the pipette against the frit.
7. Repeat step 7 using the same 10 mL pipette.
8. Centrifuge the sample at 200 g for 8 minutes at RT with the braking setting to ON to collect the cell pellet.
9. Prepare 4.5 mL of isolation buffer to a new tube for each sample.
10. Use 25 mL pipette to gently aspirate off the supernatant as much as possible without disturbing the pellet. Use a P1000 pipette to remove the remaining if too much left close to the bottom (up to 0.5 mL might be left remaining).

11. Add 200 μL of isolation buffer to the pellet and gently tap the tube on the bench clockwise a few times to loosen the pellet. Tap patiently until the pellet is completely resuspended. Gently resuspend the cells with 200 μL low-binding tip and transfer to the EDTA vacutainer.

12. Rinse the residual cells in the 50 mL conical tube with another 1 mL isolation buffer and transfer to the same vacutainer. The final volume should be approximately 4.5-5 mL.

**CTC isolation using Parsortix™**

1. Priming: Select the program PX2_P and perform according to instructions.
2. CTC isolation: select the program PX2_S26. Follow the instruction in the machine. During the isolation, shake the tube every 15 minutes to avoid the cells precipitation.
3. Tube cleaning: select the program PX2_CT2 after changing cleaning cassette. Put isolation cassette in original bag with orifice side facing up.
4. Harvest: put the isolation cassette back to the clamp and select the program PX2_H. Collect 200 μL cell suspension in 1.5 mL siliconised low retention tube.
5. Machine Clean: Select the program PX2_CT or PX2_C using cleaning cassette and PBS.

**Sample harvest and slide preparation:**

200 μL of CTC cell suspension is harvested into 1.5 mL Low-Retention microcentrifuge tube (Fisherbrand ™), centrifuge at 1,000 g for 3 min, re-suspended in 10 μL buffer (0.075 M KCl) and transfer onto slide using Superslik ™ surface and wide orifice pipette tips (VWR). The tube is rinsed with another 10 μL buffer and added to the drop on the slide. The slide is air-dried followed by fixation with acetone on ice for 20 min.

**Immunofluorescence staining:**

After blocking by 10% normal donkey serum for 10 min, cells for CTC analysis are incubated with PE-conjugated anti-CD45 (Clone: 5B1, Miltenyi Biotec) for 15 min. Cells are then permeabilised with 0.1% Triton X-100 for 5 min, stained with FITC-conjugated anti-CK (Clone: CK3-6H5, Miltenyi Biotec) and Alexa Fluor☐647-conjugated anti-VIM (Clone: EPR3776, abcam) for 30 min.

After the application of antibodies, slides are mounted in SlowFade® gold antifade mountant with DAPI. Enumeration is performed after the slide is scanned by Ariol image analysis system (Leica Microsystems (Gateshead) Ltd, UK), equipped with an Olympus BX61 microscope.

Optional: If megakaryocytes tend to be confirmed by immunostaining, a second round staining could be performed for a slide after it is scanned.

Slides are washed in 0.1% PBS-T for 2 min to remove coverslips carefully. Rinse cell area with PBS twice. Cells for megakaryocytes identification are then incubated with anti-CD41 (Clone: M148, abcam) and anti-CD34 (Clone: H-140, Santa-Cruz) for 1 h after permeabilised with 0.1% Triton X-100 for 5 min. Alexa Fluor ® 488 donkey anti-mouse and Alexa Fluor ® 546 donkey anti-rabbit (Life technologies) are then incubated with secondary antibody for 20 minutes.

**Criteria to identify CTCs and megakaryocytes:**

Enumeration:

Epithelial CTCs are defined as those CK positive, VIM negative, CD45 negative, DAPI stained intact cells; EMTing CTCs are defined as those CK positive, VIM positive, CD45 negative, DAPI stained intact cells; Mesenchymal CTCs are defined as those CK negative, VIM positive, CD45 negative, DAPI stained intact cells; Megakaryocytes are defined as cells with a strong DAPI stained nucleus $\geq$ 10 μm in size and negative for CD45, CK, and VIM staining.

Example 2 - Detection of circulating cells with epithelial and mesenchymal features

Using an optimized CTC isolation and detection method (Xu *et al.,* (2015)), blood samples from 81 prostate cancer patients (38 with untreated localized disease and 43 with CRPC) (Table 2 and Table 3) we analyzed to identify CK+/VIM-/CD45-, CK+/VIM+/CD45- and CK-/VIM+/CD45-circulating cells (Fig. 1A). Of the 38 patients with untreated localized disease, 18 (47%) patients had detectable CK+/VIM-/CD45- circulating cells, 7 (18%) patients had detectable CK+/VIM+/CD45- cells, and 21 (55%) patients had detectable CK-/VIM+/CD45- circulating cells. In the 43 with CRPC, 31 (72%), 30 (70%), and 38 (88%) patients had detectable CK+/VIM-/CD45-, CK+/VIM+/CD45-, and CK-/VIM+/CD45- circulating cells, respectively (Table 3). Of 81 patients studied, 58 (72%) patients had detectable CK+/CD45- cells regardless of VIM expression, while an additional 15 (19%) patients had no detectable CK+/CD45- cells but detectable CK-/VIM+/CD45-circulating cells.

Table 2. Clinical characteristics and CTC/megakaryocyte cell count for all patients

| Patient ID | Age | Primary GS | PSA (ng/mL) | ALP (U/L) | LDH (U/L) | CRPC (Y/N) | Prior treatment before sample collection | Metastasis (Y/N) | CK+/VIM- cell (n) | CK+/VIM+ cell (n) | CK-/VIM+ cell (n) | Megakaryocytes (n) |
|---|---|---|---|---|---|---|---|---|---|---|---|---|
| PC27 | 70 | 4+5 | 1574 | 54 | 367 | Yes | Dexamethasone | Yes | 3 | 0 | 9 | 1 |
| PC29 | 78 | 4+3 | 736 | 46 | 391 | Yes | cabazitaxel | Yes | 4 | 3 | 19 | 23 |
| PC17b | 67 | 4+5 | 4626 | 1277 | 656 | Yes | Cisplatin, Etoposide | Yes | 0 | 2 | 6 | 2 |
| PC33 | 60 | 5+5 | 39 | 337 | 366 | Yes | Dexamethasone | Yes | 0 | 0 | 5 | 4 |
| PC32c | 69 | 5+4 | 965 | 730 | 686 | Yes | Dexamethasone, Evorel | Yes | 33 | 20 | 53 | 7 |
| PC36 | 80 | 4+5 | 260 | 76 | 407 | Yes | Docetaxel | Yes | 13 | 5 | 8 | 2 |
| PC39 | 80 | 3+4 | 12 | 170 | 439 | Yes | ADT | Yes | 2 | 5 | 2 | 5 |
| PC40 | 58 | 4+4 | 22 | n/a | n/a | No | None | None | 2 | 26 | 5 | 3 |
| PC22b | 81 | 3+3 | 6000 | 1044 | 1197 | Yes | Diethylstilbestrol | Yes | 4 | 10 | 0 | 1 |
| PC41 | 69 | 4+5 | 39 | 312 | 756 | Yes | ADT | Yes | 5 | 13 | 1 | 0 |
| PC42 | 84 | n/a | 119 | 75 | 421 | Yes | Diethylstilbestrol | None | 0 | 7 | 14 | 3 |
| PC21b | 66 | n/a | 313 | 327 | 343 | Yes | Diethylstilbestrol | Yes | 3 | 4 | 2 | 0 |
| PC43 | 63 | 4+3 | 16 | 104 | 448 | Yes | Dexamethasone | None | 9 | 1 | 4 | 3 |
| PC44 | 83 | 3+5 | 633 | 46 | 391 | Yes | Diethylstilbestrol | Yes | 24 | 0 | 17 | 2 |
| PC45 | 84 | 3+3 | 1 | 79 | 447 | Yes | Triamcinolone | Yes | 2 | 3 | 2 | 3 |
| PC46 | 56 | 4+3 | 15 | n/a | n/a | No | None | None | 4 | 0 | 0 | 20 |
| PC7d | 60 | 4+5 | 693 | 79 | 489 | Yes | Triamcinolone | Yes | 20 | 4 | 5 | 1 |
| PC47 | 76 | 4+4 | 2 | 66 | 282 | Yes | Dexamethasone | Yes | 7 | 1 | 2 | 0 |
| PC49 | 88 | 4+3 | 1027 | 1339 | 774 | Yes | Dexamethasone | Yes | 1 | 0 | 13 | 2 |
| PC50 | 76 | n/a | 160 | 62 | 375 | Yes | CL56 | Yes | 20 | 2 | 1 | 1 |
| PC51 | 67 | n/a | 43 | 79 | 347 | Yes | ADT | Yes | 1 | 2 | 11 | 2 |
| PC52 | 81 | n/a | 77 | 66 | 276 | Yes | Diethylstil bestrol, Dexamethasone | Yes | 9 | 0 | 0 | 1 |
| PC19b | 73 | n/a | 40 | 93 | 329 | Yes | Enzalutamide | Yes | 1 | 2 | 8 | 3 |
| PC54 | 86 | 5+3 | 29 | 61 | 354 | Yes | Dexamethasone | Yes | 18 | 10 | 2 | 0 |
| PC56 | 62 | 5+5 | 9 | 181 | 413 | Yes | ADT + Radiotherapy | Yes | 3 | 3 | 6 | 1 |
| PC58 | 80 | n/a | 5 | 83 | 409 | Yes | Cisplatin, Etoposide | Yes | 0 | 9 | 2 | 3 |
| PC59 | 69 | 4+4 | 60 | 958 | 499 | Yes | Evorel, Triamcinolone | Yes | 23 | 2 | 0 | 1 |
| PC57b | 60 | n/a | 61 | 239 | 479 | Yes | Dexamethasone | Yes | 13 | 1 | 0 | 15 |

| Patient ID | Age | Primary GS | PSA (ng/mL) | ALP (U/L) | LDH (U/L) | CRPC (Y/N) | Prior treatment before sample collection | Metastasis (Y/N) | CK+/VIM- cell (n) | CK+/VIM+ cell (n) | CK-/VIM+ cell (n) | Megakaryocytes (n) |
|---|---|---|---|---|---|---|---|---|---|---|---|---|
| PC60 | 77 | 4+4 | 11 | 72 | 328 | Yes | Enzalutamide | Yes | 0 | 5 | 11 | 0 |
| PC61 | 70 | n/a | 22 | 50 | 349 | Yes | ADT | Yes | 1 | 2 | 7 | 0 |
| PC16c | 82 | n/a | 392 | 409 | 443 | Yes | Diethylstilbestrol | Yes | 2 | 1 | 1 | 6 |
| PC63 | 74 | n/a | 550 | 766 | 509 | Yes | Dexamethasone, Evorel | Yes | 1 | 0 | 2 | 6 |
| PC65 | 87 | n/a | 107 | 326 | 391 | Yes | ADT withdrawl | Yes | 4 | 2 | 5 | 1 |
| PC66 | 58 | 3+3 | 5 | n/a | n/a | No | None | None | 0 | 0 | 2 | 3 |
| PC69 | 67 | 5+5 | 4 | 131 | 374 | Yes | ADT | Yes | 3 | 4 | 3 | 0 |
| PC68 | 73 | 4+3 | 5 | 82 | 407 | Yes | ADT | Yes | 4 | 2 | 0 | 3 |
| PC71 | 65 | 4+4 | 37 | 76 | 406 | Yes | Docetaxel | Yes | 0 | 3 | 4 | 3 |
| PC73 | 67 | 3+4 | 12 | n/a | n/a | No | None | None | 0 | 0 | 1 | 0 |
| PC72 | 74 | 4+5 | 23 | 203 | 324 | Yes | ADT | Yes | 0 | 0 | 1 | 4 |
| PC75 | 63 | 3+3 | 5 | n/a | n/a | No | None | None | 1 | 0 | 1 | 3 |
| PC77 | 66 | 3+3 | 12 | n/a | n/a | No | None | None | 0 | 0 | 1 | 2 |
| PC80 | 62 | 4+5 | 26 | n/a | n/a | No | None | None | 5 | 0 | 4 | 2 |
| PC81 | 69 | 3+3 | 4 | n/a | n/a | No | None | None | 0 | 0 | 4 | 0 |
| PC82 | 66 | 3+3 | 6 | n/a | n/a | No | None | None | 1 | 0 | 5 | 0 |
| PC83 | 76 | 3+4 | 10 | n/a | n/a | No | None | None | 0 | 0 | 0 | 0 |
| PC84 | 56 | 3+3 | 45 | n/a | n/a | No | None | None | 0 | 6 | 4 | 3 |
| PC85 | 69 | 3+3 | 8 | n/a | n/a | No | None | None | 0 | 0 | 0 | 0 |
| PC92 | 34 | 3+3 | 4 | n/a | n/a | No | None | None | 0 | 0 | 0 | 8 |
| PC93 | 75 | 4+3 | 8 | n/a | n/a | No | None | None | 4 | 0 | 0 | 0 |
| PC96 | 73 | 3+4 | 5 | n/a | n/a | No | None | None | 2 | 0 | 0 | 3 |
| PC100 | 76 | 4+3 | 10 | n/a | n/a | No | None | None | 0 | 0 | 0 | 7 |
| PC99 | 77 | 4+3 | 9 | n/a | n/a | No | None | None | 0 | 0 | 0 | 0 |
| PC101 | 70 | 3+4 | 3 | n/a | n/a | No | None | None | 4 | 0 | 0 | 0 |
| PC103 | 71 | 3+4 | 9 | n/a | n/a | No | None | None | 2 | 0 | 0 | 1 |
| PC106 | 50 | 3+3 | 5 | n/a | n/a | No | None | None | 0 | 0 | 0 | 2 |
| PC107 | 73 | 4+3 | 7 | n/a | n/a | No | None | None | 0 | 0 | 3 | 6 |
| PC108 | 72 | 3+4 | 13 | n/a | n/a | No | None | None | 0 | 0 | 0 | 0 |
| PC111 | 55 | 4+3 | 5 | n/a | n/a | No | None | None | 0 | 0 | 6 | 3 |

| Patient ID | Age | Primary GS | PSA (ng/mL) | ALP (U/L) | LDH (U/L) | CRPC (Y/N) | Prior treatment before sample collection | Metastasis (Y/N) | CK+/VIM- cell (n) | CK+/VIM+ cell (n) | CK-/VIM+ cell (n) | Megakaryocytes (n) |
|---|---|---|---|---|---|---|---|---|---|---|---|---|
| PC115 | 67 | 4+3 | 20 | n/a | n/a | No | None | None | 2 | 1 | 3 | 2 |
| PC116 | 57 | 3+4 | 12 | n/a | n/a | No | None | None | 1 | 0 | 0 | 0 |
| PC118 | 57 | 4+3 | 9 | n/a | n/a | No | None | None | 3 | 0 | 0 | 1 |
| PC119 | 71 | 3+4 | 6 | n/a | n/a | No | None | None | 0 | 1 | 2 | 5 |
| PC55 | 58 | 3+3 | 5 | n/a | n/a | No | None | None | 1 | 1 | 1 | 7 |
| PC79 | 55 | 3+4 | 7 | n/a | n/a | No | None | None | 0 | 0 | 1 | 9 |
| PC114 | 71 | 4+5 | 49 | n/a | n/a | Yes | | Yes | 2 | 0 | 6 | 1 |
| PC104 | 76 | 4+3 | 220 | 373 | n/a | Yes | | Yes | 1 | 0 | 2 | 2 |
| PC105 | 55 | 4+5 | 52 | 81 | 393 | Yes | | Yes | 0 | 0 | 4 | 0 |
| PC144 | 71 | 3+4 | 65 | 69 | 372 | Yes | | Yes | 0 | 0 | 1 | 5 |
| PC125 | 83 | n/a | 324 | 135 | 341 | Yes | | Yes | 100 | 0 | 3 | 2 |
| PC122 | 78 | 3+4 | 14 | n/a | n/a | No | None | None | 0 | 0 | 0 | 0 |
| PC123 | 72 | 3+4 | 11 | n/a | n/a | No | None | None | 2 | 1 | 0 | 9 |
| PC124 | 69 | 4+4 | 20 | n/a | n/a | No | None | None | 2 | 0 | 1 | 0 |
| PC126 | 54 | 5+4 | 13 | n/a | n/a | No | None | None | 3 | 3 | 12 | 4 |
| PC127 | 66 | 3+4 | 367 | 62 | 317 | Yes | ADT | None | 0 | 0 | 13 | 1 |
| PC128 | 69 | 3+4 | 8 | n/a | n/a | No | None | None | 0 | 0 | 6 | 1 |
| PC129 | 84 | 4+5 | 189 | 73 | 472 | Yes | | Yes | 0 | 1 | 4 | 2 |
| PC130 | 56 | 3+4 | 10 | n/a | n/a | No | None | None | 4 | 0 | 0 | 5 |
| PC131 | 57 | 4+3 | 11 | n/a | n/a | No | None | None | 0 | 0 | 20 | 1 |
| PC132 | 61 | >7 | 50 | 59 | 344 | Yes | | Yes | 0 | 1 | 6 | 1 |
| PC135 | 55 | 3+3 | 5 | n/a | n/a | No | None | None | 0 | 0 | 2 | 3 |
| PC136 | 73 | 3+4 | 8 | n/a | n/a | No | None | None | 3 | 0 | 15 | 3 |

GS: Gleason score; PSA: prostate specific antigen; ALP: alkaline phosphatase; LDH: lactate dehydrogenase; megakaryocytes: CK-/VIM-/CD45- cells with big nuclei (see main text); ADT: androgen deprivation therapy; CL56: refers to chlorambucil + lomustine; n/a: data not available.

Table 3. Summary of Clinical characteristics and CTC count for all patients

| | All patients, n = 81 | Patient with untreated localized disease, n = 38 | Patient with CRPC, n = 43 |
|---|---|---|---|
| Age, y | | | |
| Median (IQR) | 69 (76-62) | 66.5 (72-57) | 73 (81-67) |
| PSA, ng/mL | | | |
| Median (IQR) | 15 (71-7.65) | 8.91 (12.25-5.40) | 61 (367-23) |
| Primary Gleason Score, n (%) | | | |
| 6 | 13 (16) | 11 (29) | 2 (5) |
| 3+4 | 17 (21) | 14 (37) | 3 (7) |
| 4+3 | 14 (17) | 9 (24) | 5 (12) |
| 8-10 | 24 (30) | 4 (10) | 20 (46) |
| n/a | 13 (16) | 0 (0) | 13 (30) |
| Prior therapy, n (%) | | | |
| No treatment | 38 (47) | 38(100) | 0(0) |
| Systemic therapy | 43 (53) | 0(0) | 43 (100) |
| Metastasis, n (%) | | | |
| No | 41 (51) | 38 (100) | 3 (7) |
| Yes | 40 (49) | 0 (0) | 40 (93) |
| CK+/VIM-/CD45- cell | | | |
| Detectable patient number (%) | 49 (60) | 18 (47) | 31 (72) |
| Cell number, median (IQR) | 1 (4-0) | 0 (2-0) | 2 (9-0) |
| CK+/VIM+/CD45- cell | | | |
| Detectable patient number (%) | 37 (46) | 7 (18) | 30 (70) |
| Cell number, median (IQR) | 0 (2-0) | 0 (0-0) | 2 (4-0) |
| CK-/VIM+/CD45-cell | | | |
| Detectable patient number (%) | 59 (73) | 21 (55) | 38 (88) |
| Cell number, median (IQR) | 2 (6-0) | 1 (4-0) | 4 (8-2) |
| Total CTC | | | |
| Detectable patient number (%) | 73 (90) | 30 (79) | 43 (100) |
| Cell number, median (IQR) | 6 (14-3) | 3 (6-1) | 11 (19-7) |

IQR: interquartile range (Q75-Q25%); CRPC: castration-resistant prostate cancer; CK: cytokeratin; VIM: Vimentin.

Example 3 - Genetic evidence that CK-/VIM+/CD45- circulating cells are malignant cells with genomic alterations

Multiple FISH analysis after immunofluorescence staining was firstly tested using slides with lymphocytes spiked with PC3 cells. It was found that the immunofluorescence signals were completely removed by the stripping buffer, but not completely removed by 2XSSC buffer, fix solution or proteinase K digestion. The poly-lysine coating time length of the slides was further optimized to 45 minutes to best preserve cells for downstream FISH after signal striping. Less coating time frequently resulted in damaged or lost cells after signal striping. Using these optimized conditions, we detected clear nuclear morphology and FISH signals in up to the fifth round of FISH on cells after immunofluorescence (data not shown). The technique was then successfully applied to 12 prostate cancer CTC cases for five rounds of FISH (Fig. 1B) and one case for two rounds due to strong florescence background, to investigate the genetic alterations of nine genes.

Changes of more than 30% of the genomic regions were detected in 68% of CK+/VIM-/CD45- cells, 57% of CK+/VIM+/CD45- cells and 54% of CK-/VIM+/CD45- cells, while only detected in 3.7% of CK-/VIM-/CD45+ lymphocytes and 7.8% of CK-/VIM-/CD45- cells (Table 4). The similar rate of genetic changes in the CK+/VIM-/CD45-, CK+/VIM+/CD45- and CK-/VIM+/CD45- circulating cells indicates that the majority of CK-/VIM+/CD45- cells were CTCs.

While it is possible that small proportions of the above three circulating cell categories are of non-malignant origin, we considered all CK+/VIM-/CD45-, CK+/VIM+/CD45- and CK-/VIM+/CD45- cells for the correlation analysis between CTC numbers and clinical features, and categorized them as epithelial, EMTing or mesenchymal CTCs, respectively. When classifying cases as positive or negative for CTCs, the number of CK+/VIM-/CD45-, CK+/VIM+/CD45- and CK-/VIM+/CD45- circulating cells found in non-cancer healthy control cases were considered.

Table 4. Proportion of detected genetic changes in sub-populations of cells

| Subtypes of CTCs | 0%[a] n (%)[b] | <30% n (%) | 30-49% n (%) | 50-69% n (%) | 70-99% n (%) | 100% n (%) |
|---|---|---|---|---|---|---|
| Epithelial (CK+/VIM-/CD45-), (n=25) | 4 (16) | 4 (16) | 5 (20) | 7 (28) | 1 (4) | 4 (16) |
| EMTing (CK+/VIM+/CD45-), (n=39) | 11 (28) | 6 (15) | 9 (23) | 8 (21) | 2 (5) | 3 (8) |
| Mesenchymal (CK-/VIM+/CD45-), (n=54) | 8 (15) | 17 (31) | 17 (31) | 9 (17) | 2 (4) | 1 (2) |
| Lymphocytes (CK-/VIM-/CD45+), n=134 | 81 (60.4) | 48 (35.8) | 5 (3.7) | 0 (0) | 0 (0) | 0 (0) |
| Negative cells (CK-/VIM-/CD45-), n=102 | 66 (64.7) | 28 (27.5) | 5 (4.9) | 3 (2.9) | 0 (0) | 0 (0) |

[a]The proportion of abnormal probes of the total countable probes in a cell (only with > 2 genomic region informative was included).

[b]The number and percentage of cells in each category of detected circulating cells with certain frequency of genomic alterations.

Example 4 - The presence of circulating megakaryocytes was associated with better survival

Unexpectedly, a rare population of cells was found in the harvested samples, with big nuclei (larger than both lymphocytes and most CTCs) of strong DAPI staining, but negative for CK, VIM and CD45 (called megakaryocytes below) (Fig. 2A). Due to the strong DAPI staining and larger size of nuclei, such cells were easily identified from other cells with DAPI staining alone (Fig. 6), though lack of CK, VIM and CD45 staining helped further excluding possible CTCs and large lymphocytes. FISH analysis for these cells showed 100% polyploidy and most (61.9%) had more than ten copies of the genome in each cell (Fig. 3A), suggesting a special type of cells, distinct to lymphocytes or CTCs. The mean nuclear diameter of megakaryocytes was $17.5 \pm 4.9$ $\mu$m (range: 10 - 32 $\mu$m), while the mean nuclear diameter for lymphocytes was $6.5 \pm 0.8$ $\mu$m (range: 4 - 8 $\mu$m). No nuclear size overlap was observed between these two categories (Fig. 7 - note BigNeg refers to megakaryocytes). Therefore, megakaryocytes were distinguishable from other cells just through immunostaining and size measurement using the following criteria: cells with a strong DAPI stained nucleus $\geq$ 10 $\mu$m in size and negative for CD45, CK, and VIM staining. Using these criteria, such cells were counted in all 81 blood samples. The numbers were variable and lower in CRPC patients died during follow-up (median 1 cell/7.5 mL, IQR: 2-1 cells/7.5 mL) than those CRPC patients still alive (median 2 cells/7.5 mL, IQR: 3.75-1 cells/7.5 mL) and also than patients with untreated localized disease (median 2 cells/7.5 mL, IQR: 4.25-0 cells/7.5 mL), though none of the differences were significant (p value of 0.14 and 0.33, respectively). In the 43 CRPC patients, high megakaryocyte count was associated with better survival (HR: 0.836, p = 0.27, Table 3), although statistical significance was not achieved. Nevertheless, the estimated survival rates were significantly shorter in those with less than three megakaryocytes by Kaplan-Meier curve (p = 0.021, Fig. 2B).

Based on previous results that high mesenchymal CTC counts generally represent more aggressive tumors and are associated with a higher risk for death, we hypothesize that the difference between the number of mesenchymal CTCs and megakaryocytes may further enhance the ability to predict survival. This was confirmed by univariate survival analysis model (HR: 1.059, p = 0.004, Table 5) and by Kaplan-Meier survival analysis where a difference of $\geq$4 cells most significantly separates different survival groups (p = 0.0007, Fig. 4).

Table 5. Predictors for survivals in progressive prostate cancer at baseline

| | HR (95% CI) | $\chi^{12}$ (p) |
|---|---|---|
| Age (y) | 0.996 (0.927-1.071) | 0.01 (0.92) |
| PSA (ng/mL) | 1.0006 (1.0003-1.0009) | 10.23 (<0.001) |

(continued)

| | HR (95% CI) | $\chi_1^2$ (p) |
|---|---|---|
| Primary GS | 1.235 (0.648-2.354) | 0.42 (0.52) |
| ALP (U/L) | 1.003 (1.001-1.003) | 9.58 (0.001) |
| LDH (U/L) | 1.004 (1.001-1.006) | 6.58 (0.002) |
| Epithelial CTCs (n) | 1.006 (0.974-1.039) | 0.12 (0.71) |
| EMTing CTCs (n) | 1.067 (0.948-1.202) | 0.98 (0.28) |
| Mesenchymal CTCs (n) | 1.046 (1.007-1.088) | 3.53 (0.022) |
| Total CK+ CTCs (n) | 1.009 (0.980-1.039) | 0.32 (0.54) |
| Total VIM+ CTCs (n) | 1.033 (1.003-1.063) | 3.05 (0.032) |
| Total CTCs (n) | 1.015 (0.995-1.035) | 1.65 (0.14) |
| Megakaryocytes (n) | 0.836 (0.607-1.151) | 2.01 (0.27) |
| Mesenchymal CTCs minus megakaryocytes | 1.059 (1.018-1.101) | 5.59 (0.004) |

HR: hazard ratio; CI: confidential interval; ALP: alkaline phosphatase; LDH: lactate dehydrogenase; megakaryocytes: CK-/VIM-/CD45- cells with big nuclei (see main text below).

In the 40 metastatic CRPC (mCRCR) patients, high megakaryocyte cell count had a trend to be associated with better survival (HR, 0.849; 95% CI, 0.628-1.146; $P_{adj}$ = 0.31; Table 6. When a cutoff was selected, patients with megakaryocyte (≥3 cells) had an HR of 0.144 (95% CI, 0.018-1.129; P = 0.02). The estimated survival rates were significantly higher in those with ≥3 megakaryocyte cells by Kaplan-Meier curve (P = 0.03, Fig. 8A). Note "BigNeg" in Fig 8A refers to megakaryocytes.

In addition, using the formula [(mesenchymal CTC count - megakaryocytes cell count)/ megakaryocytes cell count to calculate a combined mesenchymal CTC and megakaryocyte score (CMS, a particular CRS) and a univariate Cox model for survival analysis, the HR for per unit increase of the score is 1.282 (95% CI, 1.097-1.499; $P_{adj}$ = 0.015); Table 6. When a cutoff was selected, patients with CMS (2.0) had an HR of 10.170 (95% CI, 2.164-47.789; P = 0.0005) and by Kaplan-Meier survival analysis, a CMS 2.0 cutoff most significantly separated different survival groups (P = 0.0003; Fig. 8B). Note "BigNeg" in Fig 8B refers to megakaryocytes.

Table 6. Predictors for survival in progressive mCRPC patients at baseline

| | HR (95% CI) | LR $\chi^2$ test | $P_{adj}$[a] |
|---|---|---|---|
| Age (y) | 0.994 (0.923-1.070) | 0.02 | 0.875 |
| PSA (ng/mL) | 1.0006 (1.0003-1.0009) | 9.84 | 0.015 |
| GS | 1.145 (0.598-2.193) | 0.17 | 0.804 |
| ALP (U/L) | 1.003 (1.001-1.004) | 8.77 | 0.015 |
| LDH (U/L) | 1.003 (1.001-1.006) | 6.28 | 0.040 |
| Epithelial CTCs (n) | 1.005 (0.972-1.039) | 0.08 | 0.847 |
| EMTing CTCs (n) | 1.066 (0.948-1.198) | 0.95 | 0.477 |
| Mesenchymal CTCs (n) | 1.046 (1.008-1.086) | 3.78 | 0.135 |
| Total CK+ CTCs (n) | 1.008 (0.979-1.038) | 0.24 | 0.804 |
| Total VIM+ CTCs (n) | 1.032 (1.003-1.062) | 3.21 | 0.158 |
| Total CTCs (n) | 1.014 (0.995-1.035) | 1.58 | 0.338 |
| Megakaryocytes (n) | 0.849 (0.628-1.146) | 1.93 | 0.306 |
| CMS | 1.282 (1.097-1.499) | 8.59 | 0.015 |

Abbreviations: ALP, alkaline phosphatase; Megakaryocytes, CK-/VIM-/CD45- cells with big nuclei; GS, Gleason score of the primary tumor; LDH, lactate dehydrogenase.
[a]Benjamini and Hochberg adjustment (Benjamini & Hochberg, J R Stat Soc Series B Methodol, 1995, 57:289-300)

In blood samples from the 12 healthy donors, the median number of megakaryocytes was 1 cell/7.5 mL (range: 0-3, IQR: 1-0, Fig. 5), and in a study using a larger sample of healthy donors, blood samples from 24 healthy donors, the median number of megakaryocytes cells was 1 cell/7.5 mL (range, 0-15; IQR, 2.5-0). These were both marginally lower than that in the 38 patients with untreated localized disease (median: 2, range: 0-20, IQR: 4.25-0, Fig. 5) ($x_1^2$(p) = 3.651(0.056)) and significantly lower than that in 43 CRPC patients (median: 2, range: 0-23, IQR: 3-1, Fig. 5) ($x_1^2$(p)

= 5.993(0.014)).

Finally, the nature of the megakaryocytes was determined. Their consistent hyperploidy revealed by FISH analysis highly suggested a potential megakaryocyte-like nature. We established the megakaryocytes immunofluorescence analysis method by detecting CD34 and CD41 expression in megakaryocytes induced by phorbol myristate acetate (PMA) from K562 cells (Fig. 3B). All megakaryocytes were CD34 positive and 92% (101/110) of them were CD41 positive, confirming them as megakaryocytes (Fig. 3C). CD45+ lymphocytes, epithelial CTCs, EMTing CTCs, and mesenchymal CTCs were all negative for CD34 and CD41.

The most striking finding is the unexpected association of circulating megakaryocytes in progressed disease with patient survival, which indicates that these megakaryocytes play a role in inhibiting cancer cells. In addition to the bone marrow and the spleen, megakaryocytes have also been detected at very low numbers in circulation (Psaila et al., J Thromb Haemost 10, 177-188 (2012); Bojko et al., J Clin Apher 13, 7-15 (1998)). Megakaryocytes experience a life cycle from a proliferative 2n stage to the process of polyploidization, accumulating a DNA content of 8n, 16, 32n, 62n and even 128n before their final maturation and platelet fragmentation (Zimmet et al., Exp Hematol 28, 3-16 (2000)). Increased ploidization of megakaryocytes has been reported in patients with metastatic tumors (Winkelmann et al., Haemostasis 14, 501-507 (1984)). The circulating megakaryocytes detected in this study with high DNA content and consistent expression of the early development biomarker CD34 represent a subgroup of megakaryocytes, which are in the middle of maturation. The association of low number of megakaryocytes with a poorer survival of CRPC patients suggests an anti-tumor effect of megakaryocytes. To support this, megakaryocytes have been reported to increase their number in response to the presence of tumor in mouse models (Zaslavsky, et al., Blood 115, 4605-4613 (2010); Li *et al.,* (2011)) and inhibit the proliferation of prostate cancer cell lines when co-cultured (Li *et al.,* (2011)).

As the nuclei of megakaryocytes are big and with strong DAPI staining, they can be easily identified during CTC analysis. A group of large hyperploid cells with no detectable biomarkers (including epithelial and hepatocellular cancer specific markers) have also been reported recently (Ogle et al., J Hepatol, (2016)). As the authors did not include any mesenchymal markers, they considered all these cells as CTCs with EMT. Based on this study, these cells might be a mixture of mesenchymal CTCs and circulating megakaryocytes. Megakaryocytes have also been identified in a previous CTC study of prostate cancer, but the investigators exclude them for further analysis (Leversha *et al.,* (2009)). Taking advantage of the size and/or deformability based CTC isolation platform, such cells can be captured with high efficiency for cancer prognosis.

Here, we not only showed that mesenchymal CTCs has the potential to be better associated with patient survival than CTCs with epithelial features, but also found that the combination of mesenchymal CTC and megakaryocyte counts has a great power to predict CRPC patient survival, with 1.28-fold risk of death per unit increase of the CMS and 10-fold risk for patients with a score ≥ 2.0.

Accordingly, circulating megakaryocytes represent an efficient biomarker for survival prediction for patients with CRPC.

Materials and Methods

**Patients and blood samples**

A total of 81 patients with written consent were recruited from December 2014 in St Bartholomew's Hospital, Barts Health NHS, London, UK, comprising 38 with untreated localized prostate cancer, and 43 with progressive CRPC (40 with metastasis) ready to commence an alternative treatment. Metastases were investigated by radionuclide bone scan and computed tomography (CT). Progressive diseases were defined with a minimum of two increasing PSA levels at least two weeks apart or by radiographic criteria new lesions by bone scan or as new or enlarged soft tissue by CT or magnetic resonance imaging. Blood specimens from 12 healthy donors were collected with signed Ethics committee approved consent form. 7.5mL of whole blood was donated from each participant for CTC enumeration. Blood samples were drawn into EDTA Vacutainer tubes (Becton Dickinson and Company, Polymouth, UK) and acquired at the middle of phlebotomy after the collection for routine clinical blood test to avoid contamination with epithelial cells from the skin. All blood samples were stored at room temperature and processed within 24 hours of blood draw. The clinical characteristics of the above 81 patients are detailed in Table 2 and Table 3.

**Cell culture**

The human prostate cancer cell line, PC3, was used to simulate cancer samples as a validation test for repeated FISH after immunofluorescence before performing in clinical samples. Megakaryocyte cell lines were obtained by treating human hematopoietic precursor cells K562 (a patient-derived leukemia cell line which expresses both erythroid and MK markers and can be induced to differentiate). K562 cells were cultured in Iscove's Modified Dulbecco's Media (Sigma Aldrich) + 10% fetal bovine serum (Gibco, Life technologies) and induced with 50 nM of phorbol myristate acetate (PMA) (Sigma Aldrich) to differentiate into mature MKs. This was used as validation for CD34 and CD41 immunostaining. All the cell lines are from ATCC.

## CTC harvest and fixation on slides

200 μL of CTC cell suspension isolated from 7.5 ml blood samples using Parsortix system was harvested into 1.5 mL Low-Retention microcentrifuge tube (FisherbrandTM), centrifuge at 1,000 g for 3 min, re-suspended in 10 μL buffer (0.075M KCl) and transfer onto slide using SuperslikTM surface and wide orifice pipette tips (VWR). The tube was rinsed with another 10 μL of buffer and added to the drop on the slide. The slide was air-dried followed by fixation with acetone on ice for 20 min.

## Immunofluorescence staining

After blocking by 10% normal donkey serum for 10 min, cells for CTC analysis were incubated with PE-conjugated anti-CD45 (Clone: 5B1, Miltenyi Biotec) for 15 min. Cells were then permeabilized with 0.1% Triton X-100 for 5 min, stained with FITC-conjugated anti-CK (Clone: CK3-6H5, Miltenyi Biotec) and Alexa Fluorâ647-conjugated anti-VIM (Clone: EPR3776, abcam) for 30 min.

Cells for megakaryocytes identification were incubated with anti-CD41 (Clone: M148, abcam) and anti-CD34 (Clone: H-140, Santa-cruz) for 1 h after permeabilized with 0.1% Triton X-100 for 5 min. Alexa Fluorâ488 donkey anti-mouse and Alexa Fluorâ546 donkey anti-rabbit (Life technologies) secondary antibodies were then incubated with the cells for 20 minutes. After the application of antibodies, slides were mounted in SlowFade® gold antifade mountant with DAPI. Enumeration was performed after the slide was scanned by Ariol image analysis system (Leica Microsystems (Gateshead) Ltd, UK), equipped with an Olympus BX61 microscope.

## Multiple FISH procedure

FISH analysis was performed for AR (RP11-479J1), *PTEN* (CTD-846G17), ERG (RP11-476D17 and RP11-95I21), *TMPRSS2* (RP11-535H11), *NXK3.1* (RP11-213G6), *C-MYC* (RP11-349C2), *RB1* (RP11-305D15 and RP11-174I10), *CCND1* (RP11-278A17, RP11-599F23, RP11-681H17 and CTD-2009H2), 6q16 (RP11-639P13, RP11-258I9, CTD-2281M23 and CTD-2073M5), 16q22.1 (RP11-510M2), chromosome 1 centromere, *RAF1* 3' (RP11-64E16, RP11-136B7 and RP11-738A2) and *RAF1* 5' (RP11-715I4, RP11-764F12 and RP11-449E21). Chromosome1 centromere, *RAF1* 3' and *RAF1* 5' were from Institute of Cancer Research. All other FISH probes were purchased from Life Technologies (UK). Probes were prepared as previously described (39). Probes for *PTEN,* RP11-95I21, *TMPRSS2, NKX3.1,* 6q16, 16q22.1 and *RAF1* 5' were labeled by Fluorescein-12-dUTP (Roche, IN, USA). Probes for *AR*, RP11-476D17, *C-MYC, RB1, CCND1* and *RAF1* 3' were labeled by Tetramethyl-rhodamine-5-dUTP (Roche, IN, USA). Before hybridization, slides were fixed in methanol: acetic acid (3:1) for 10 minutes, and then pretreated in 70% acetic acid in 10 minutes. Approximately 100 ng of each of the labeled clones were resuspended in hybridization buffer. The mixture was applied to the slide, denatured at 95°C for 10 minutes and incubated at 37°C overnight in a wet box. The slide was washed following the standard FISH method.

Two FISH probes were hybridized with the slide in each round of FISH. To apply new FISH probes, the FISH signal was removed in 70% formamide/2XSSC solution at 68°C for 4 minutes, followed by rinsing in 2XSSC and water. After being air dried, the slide was ready to hybridize with the new pair of FISH probes.

After mounting with antifade DAPI, the FISH signals were scanned by Ariol (Leica Microsystems (Gateshead) Ltd, UK).

## Statistics

Unless specifically noted, Wilcoxon rank-sum test was applied to assess the equality of CTCs between subgroups based on CTC-score as well as different clinical features, such as metastasis, primary GS, and risk classification in localized disease. Data shown was as median (IQR). Spearman's rank correlation was used to assess the association between CTC counts and concurrent PSA level. ROC curve analysis was used to test the ability of different subtypes CTCs as well as combined risk score (CRS) to distinguish patients with metastasis. PSA score and EMTing CTC count were combined in a logistic regression. The outcome was metastasis (yes, no). The coefficients obtained from this model were used to compute CRS. Optimal cut-off point was calculated with an optimal corrected classified value to provide best available sensitivity and specificity. Rocgold function was used to independently test the equality of the ROC area of each method against the PSA as a gold standard curve. The significance of the association of genomic alteration and clinical features were performed using Barnard's Test. To determine the factors that predict survival time, variables considered as potential predictors were selected for univariate analyses using Cox model. Survival curves were generated using the Kaplan-Meier method and compared using the log-rank test. All statistical tests will be two sided and p-values of 0.05 will be accepted as statistically significant. No adjustment for multiple comparisons was performed. Statistical analyses were performed using Stata 13.0.

**Claims**

1. A method of determining prognosis of prostate cancer in a subject comprising measuring the amount of megakaryocytes in a blood sample from the subject.

2. The method of claim 1, wherein less than or equal to a threshold amount of megakaryocytes indicates a poor prognosis, optionally wherein less than 3 megakaryocytes per 7.5 mL of sample indicates a poor prognosis.

3. The method of claim 1 or claim 2, wherein:

   the megakaryocytes are CD34 positive, CD41 positive, CD45 negative, cytokeratin (CK) negative and/or vimentin negative;
   the megakaryocytes are CD45 negative, CK negative and vimentin negative;
   the megakaryocytes are CD34 positive, and/or CD41 positive, CD45 negative, CK negative and vimentin negative;
   the megakaryocytes have a nucleus with a dimension that is greater than or equal to 10 $\mu$m;
   the megakaryocytes are polyploid;
   the megakaryocytes are at least tetraploid (4N); or
   at least 50% of the megakaryocytes are at least octaploid (8N).

4. The method of any preceding claim further comprising measuring the amount of circulating tumour cells (CTCs) in the blood sample, optionally wherein:

   the CTCs are partially transitioned from epithelial CTCs to mesenchymal CTCs;
   the CTCs are CD45 negative, CK positive and/or vimentin positive;
   the CTCs are CD45 negative, CK positive and vimentin positive;
   the CTCs are mesenchymal CTCs;
   the CTCs are CD45 negative, CK negative and/or vimentin positive; or
   the CTCs are CD45 negative, CK negative and vimentin positive.

5. The method of claim 4, wherein greater than or equal to a threshold amount of CTCs indicates a poor prognosis, optionally wherein the threshold amount of CTCs per 7.5 mL of sample is selected from the group consisting of:

   (a) 10 CTCs per 7.5 mL of sample, wherein the CTCs comprise epithelial CTCs, CTCs that are partially transitioned from epithelial CTCs to mesenchymal CTCs and mesenchymal CTCs,
   (b) 7 CTCs per 7.5 mL of sample, wherein the CTCs comprise CTCs that are partially transitioned from epithelial CTCs to mesenchymal CTCs and mesenchymal CTCs, and
   (c) 5 CTCs per 7.5 mL of sample, wherein the CTCs comprise mesenchymal CTCs.

6. The method of claim 5, wherein the CTCs are mesenchymal CTCs and the prostate cancer is castration resistant prostate cancer (CRPC), and a threshold amount of CTCs is 5 CTCs per 7.5 mL of sample.

7. The method of any one of claims 4 to 6, further comprising comparing the amount of CTCs and megakaryocytes to each other, optionally wherein a difference of 4 or more cells per 7.5 mL of sample between the amount of CTCs and the amount of megakaryocytes indicates a poor prognosis, optionally wherein the CTCs are mesenchymal CTCs and/or the CTCs are CD45 negative, CK negative and/or vimentin positive.

8. The method of any preceding claim, wherein the method comprises measuring the amount of megakaryocytes and the amount of circulating tumour cells (CTCs) in the blood sample, wherein:

   the megakaryocytes are CD34 positive and/or CD41 positive, CD45 negative, CK negative and vimentin negative;
   the CTCs are CD45 negative, CK negative and vimentin positive; and

a difference of 4 or more cells per 7.5 mL of sample between the amount of CTCs and the amount of megakaryocytes indicates a poor prognosis.

9. The method of any preceding claim further comprising enriching the sample for megakaryocytes and/or CTCs, wherein the enriching is by isolating and/or counting the megakaryocytes and/or CTCs, optionally wherein the enriching, the isolating and/or the counting is by size- and/or deformability-based sorting of the megakaryocytes and/or CTCs.

10. The method of any preceding claim wherein the prostate cancer has progressed after the subject has undergone treatment for the prostate cancer.

11. The method of claim 1, wherein the prostate cancer is localised prostate cancer, advanced prostate cancer, malignant prostate cancer, aggressive prostate cancer, castration resistant prostate cancer (CRPC) or metastatic prostate cancer.

12. The method of any preceding claim further comprising measuring prostate specific antigen (PSA) concentration and/or primary Gleason score in one or more samples from the subject and/or measuring the number of metastases in the subject.

13. The method of claim 11 or claim 12, wherein the prostate cancer has been treated by hormone deprivation and the prostate cancer has one or more features selected from the group consisting of increased PSA concentration, size and number of metastases during treatment despite a serum testosterone concentration of less than 50 ng/mL, a PSA rise of 25% or more from nadir and consecutive increases in PSA in measurements taken at least three weeks apart.

14. The method of any preceding claim further comprising measuring genomic alteration in the megakaryocytes and/or the CTCs, wherein the measuring genomic alteration confirms the identity of the megakaryocytes and/or the CTCs, determines that the megakaryocytes are polyploid, or identifies one or more mutations of the CTCs and/or confirms the malignancy of the CTCs, and further optionally comprising comparing genomic alteration in the CTCs of the sample from the subject with genomic alteration in the CTCs of a control, optionally wherein the control is a sample from a healthy individual.

15. The method of claim 14, wherein the measuring genomic alteration is by fluorescence *in situ* hybridization (FISH), optionally wherein the FISH is repeated FISH, and further optionally wherein the FISH comprises stripping at 40 to 90 °C with a stripping buffer comprising 1 % to 3 % sodium dodecyl sulfate (SDS), 0.055 M to 0.075 M Tris-HCl and 0.5 % to 1.1 % $\beta$-mercaptoethanol and wherein the stripping buffer has a pH from 6.6 to 7.0.

**Patentansprüche**

1. Verfahren zum Bestimmen der Prognose von Prostatakrebs bei einem Subjekt, umfassend das Messen der Menge von Megakaryozyten in einer Blutprobe von dem Subjekt.

2. Verfahren nach Anspruch 1, wobei eine Menge kleiner oder gleich einer Schwellenmenge von Megakaryozyten eine schlechte Prognose anzeigt, wobei gegebenenfalls weniger als 3 Megakaryozyten pro 7,5 ml Probe eine schlechte Prognose anzeigt.

3. Verfahren nach Anspruch 1 oder Anspruch 2, wobei:

die Megakaryozyten CD34-positiv, CD41-positiv, CD45-negativ, Cytokeratin (CK)-negativ und/oder Vimentin-negativ sind;
die Megakaryozyten CD45-negativ, CK-negativ und Vimentin-negativ sind;
die Megakaryozyten CD34-positiv und/oder CD41-positiv, CD45-negativ, CK-negativ und Vimentin-negativ sind;
die Megakaryozyten einen Kern mit einer Abmessung von 10 $\mu$m oder mehr aufweisen;
die Megakaryozyten polyploid sind;
die Megakaryozyten mindestens tetraploid (4N) sind; oder
mindestens 50% der Megakaryozyten mindestens octaploid (8N) sind.

4. Verfahren nach einem der vorhergehenden Ansprüche, ferner umfassend das Messen der Menge von zirkulierenden Tumorzellen (CTCs) in der Blutprobe, wobei gegebenenfalls:

> die CTCs teilweise aus epithelialen CTCs zu mesenchymalen CTCs übergegangen sind;
> die CTCs CD45-negativ, CK-positiv und/oder Vimentin-positiv sind;
> die CTCs CD45-negativ, CK-positiv und Vimentin-positiv sind;
> die CTCs mesenchymale CTCs sind;
> die CTCs CD45-negativ, CK-negativ und/oder Vimentin-positiv sind; oder
> die CTCs CD45-negativ, CK-negativ und Vimentin-positiv sind.

5. Verfahren nach Anspruch 4, wobei eine Menge größer oder gleich einer Schwellenmenge von CTCs eine schlechte Prognose anzeigt, wobei gegebenenfalls die Schwellenmenge von CTCs pro 7,5 ml Probe ausgewählt ist aus der Gruppe bestehend aus:

> (a) 10 CTCs pro 7,5 ml Probe, wobei die CTCs epitheliale CTCs, CTCs, die teilweise von epithelialen CTCs zu mesenchymalen CTCs übergegangen sind, und mesenchymale CTCs umfassen,
> (b) 7 CTCs pro 7,5 ml Probe, wobei die CTCs CTCs, die teilweise von epithelialen CTCs zu mesenchymalen CTCs übergegangen sind, und mesenchymale CTCs umfassen, und
> (c) 5 CTCs pro 7,5 ml Probe, wobei die CTCs mesenchymale CTCs umfassen.

6. Verfahren nach Anspruch 5, wobei die CTCs mesenchymale CTCs sind und der Prostatakrebs kastrationsresistenter Prostatakrebs (CRPC) ist und eine Schwellenmenge von CTCs sich auf 5 CTCs pro 7,5 ml Probe beläuft.

7. Verfahren nach einem der Ansprüche 4 bis 6, ferner umfassend das Vergleichen der Menge an CTCs und Megakaryozyten miteinander, wobei gegebenenfalls ein Unterschied von 4 oder mehr Zellen pro 7,5 ml Probe zwischen der Menge an CTCs und der Menge an Megakaryozyten eine schlechte Prognose anzeigt, wobei gegebenenfalls die CTCs mesenchymale CTCs sind und/oder die CTCs CD45-negativ, CK-negativ und/oder Vimentin-positiv sind.

8. Verfahren nach jedwedem vorhergehenden Anspruch, wobei das Verfahren das Messen der Menge an Megakaryozyten und der Menge an zirkulierenden Tumorzellen (CTCs) in der Blutprobe umfasst, wobei:

> die Megakaryozyten CD34-positiv und/oder CD41-positiv, CD45-negativ, CK-negativ und Vimentin-negativ sind;
> die CTCs CD45-negativ, CK-negativ und Vimentin-positiv sind; und
> ein Unterschied von 4 oder mehr Zellen pro 7,5 ml Probe zwischen der Menge an CTCs und der Menge an Megakaryozyten eine schlechte Prognose anzeigt.

9. Verfahren nach jedwedem vorhergehenden Anspruch, ferner umfassend das Anreichern der Probe hinsichtlich Megakaryozyten und/oder CTCs, wobei das Anreichern durch Isolieren und/oder Zählen der Megakaryozyten und/oder CTCs erfolgt, wobei gegebenenfalls das Anreichern, das Isolieren und/oder das Zählen durch Sortieren der Megakaryozyten und/oder CTCs nach Größe und/oder Verformbarkeit erfolgt.

10. Verfahren nach einem der vorhergehenden Ansprüche, wobei der Prostatakrebs fortgeschritten ist, nachdem der Patient eine Behandlung für den Prostatakrebs erfahren hat.

11. Verfahren nach Anspruch 1, wobei der Prostatakrebs lokalisierter Prostatakrebs, fortgeschrittener Prostatakrebs, bösartiger Prostatakrebs, aggressiver Prostatakrebs, kastrationsresistenter Prostatakrebs (CRPC) oder metastatischer Prostatakrebs ist.

12. Verfahren nach jedwedem vorhergehenden Anspruch, ferner umfassend das Messen der Konzentration des prostataspezifischen Antigens (PSA) und/oder des primären Gleason-Scores in einer oder mehreren Proben aus dem Subjekt und/oder das Messen der Anzahl von Metastasen im Subjekt.

13. Verfahren nach Anspruch 11 oder Anspruch 12, wobei der Prostatakrebs durch Hormon-Entzug behandelt wurde und der Prostatakrebs ein oder mehrere Merkmale aufweist, die ausgewählt sind aus der Gruppe bestehend aus einer erhöhten PSA-Konzentration, Größe und Anzahl von Metastasen während der Behandlung trotz einer Serum-Testosteronkonzentration von weniger als 50 ng/ml, einem PSA-Anstieg von 25% oder mehr vom Nadir, und aufeinanderfolgenden PSA-Anstiegen bei Messungen, die mindestens drei Wochen auseinanderliegend vorgenommen werden.

14. Verfahren nach jedwedem vorhergehenden Anspruch, ferner umfassend das Messen der genomischen Veränderung in den Megakaryozyten und/oder den CTCs, wobei das Messen der genomischen Veränderung die Identität der Megakaryozyten und/oder der CTCs bestätigt, bestimmt, dass die Megakaryozyten polyploid sind, oder eine oder weitere Mutationen der CTCs identifiziert und/oder die Malignität der CTCs bestätigt, und gegebenenfalls ferner umfassend das Vergleichen der genomischen Veränderung in den CTCs der Probe von dem Subjekt mit der genomischen Veränderung in den CTCs einer Kontrolle, wobei die Kontrolle gegebenenfalls eine Probe aus einem gesunden Individuum ist.

15. Verfahren nach Anspruch 14, wobei das Messen der genomischen Veränderung durch Fluoreszenz-in-situ-Hybridisierung (FISH) erfolgt, wobei die FISH gegebenenfalls eine wiederholte FISH ist, und wobei die FISH ferner gegebenenfalls das Abstreifen (Stripping) bei 40 bis 90°C mit einem 1% bis 3% Natriumdodecylsulfat (SDS), 0,055 M bis 0,075 M Tris-HCl und 0,5% bis 1,1% $\beta$-Mercaptoethanol umfassenden Abstreifpuffer umfasst, und wobei der Abstreifpuffer einen pH-Wert von 6, 6 bis 7,0 aufweist.

**Revendications**

1. Procédé de détermination de pronostic de cancer de la prostate chez un sujet comprenant la mesure de la quantité de mégacaryocytes dans un échantillon de sang provenant du sujet.

2. Procédé selon la revendication 1, dans lequel une quantité inférieure ou égale à une quantité de mégacaryocytes de seuil indique un mauvais pronostic, éventuellement dans lequel moins de 3 mégacaryocytes pour 7,5 ml d'échantillon indique un mauvais pronostic.

3. Procédé selon la revendication 1 ou la revendication 2, dans lequel :

   les mégacaryocytes sont CD34 positifs, CD41 positifs, CD45 négatifs, cytokératine (CK) négatifs et/ou vimentine négatifs ;
   les mégacaryocytes sont CD45 négatifs, CK négatifs et vimentine négatifs ;
   les mégacaryocytes sont CD34 positifs et/ou CD41 positifs, CD45 négatifs, CK négatifs et vimentine négatifs ;
   les mégacaryocytes ont un noyau ayant une dimension qui est supérieure ou égale à 10 $\mu$m ;
   les mégacaryocytes sont polyploïdes ;
   les mégacaryocytes sont au moins tétraploïdes (4N) ; ou
   au moins 50 % des mégacaryocytes sont au moins octaploïdes (8N).

4. Procédé selon une quelconque revendication précédente comprenant en outre la mesure de la quantité de cellules tumorales circulantes (CTC) dans l'échantillon de sang, éventuellement dans lequel :

   les CTC ont en partie subi une transition de CTC épithéliales en CTC mésenchymateuses ;
   les CTC sont CD45 négatives, CK positives et/ou vimentine positives ;
   les CTC sont CD45 négatives, CK positives et vimentine positives ;
   les CTC sont des CTC mésenchymateuses ;
   les CTC sont CD45 négatives, CK négatives et/ou vimentine positives ; ou
   les CTC sont CD45 négatives, CK négatives et vimentine positives.

5. Procédé selon la revendication 4, dans lequel une quantité supérieure ou égale à une quantité de CTC de seuil indique un mauvais pronostic, éventuellement dans lequel la quantité de CTC de seuil pour 7,5 ml d'échantillon est choisie dans le groupe constitué par :

   (a) 10 CTC pour 7,5 ml d'échantillon, les CTC comprenant des CTC épithéliales, des CTC qui ont en partie subi une transition de CTC épithéliales en CTC mésenchymateuses et des CTC mésenchymateuses,
   (b) 7 CTC pour 7,5 ml d'échantillon, les CTC comprenant des CTC qui ont en partie subi une transition de CTC épithéliales en CTC mésenchymateuses et des CTC mésenchymateuses et
   (c) 5 CTC pour 7,5 ml d'échantillon, les CTC comprenant des CTC mésenchymateuses.

6. Procédé selon la revendication 5, dans lequel les CTC sont des CTC mésenchymateuses et le cancer de la prostate est un cancer de la prostate résistant à la castration (CPRC) et une quantité de CTC de seuil est de 5 CTC pour 7,5 ml d'échantillon.

**7.** Procédé selon l'une quelconque des revendications 4 à 6, comprenant en outre la comparaison de la quantité de CTC et de la quantité de mégacaryocytes l'une avec l'autre, éventuellement dans lequel une différence supérieure ou égale à 4 cellules pour 7,5 ml d'échantillon entre la quantité de CTC et la quantité de mégacaryocytes indique un mauvais pronostic, éventuellement dans lequel les CTC sont des CTC mésenchymateuses et/ou les CTC sont CD45 négatives, CK négatives et/ou vimentine positives.

**8.** Procédé selon une quelconque revendication précédente, le procédé comprenant la mesure de la quantité de mégacaryocytes et de la quantité de cellules tumorales circulantes (CTC) dans l'échantillon de sang, dans lequel :

les mégacaryocytes sont CD34 positifs et/ou CD41 positifs, CD45 négatifs, CK négatifs et vimentine négatifs ;
les CTC sont CD45 négatives, CK négatives et vimentine positives ; et
une différence supérieure ou égale à 4 cellules pour 7,5 ml d'échantillon entre la quantité de CTC et la quantité de mégacaryocytes indique un mauvais pronostic.

**9.** Procédé selon une quelconque revendication précédente comprenant en outre l'enrichissement de l'échantillon en mégacaryocytes et/ou en CTC, dans lequel l'enrichissement se fait par isolement et/ou numération des mégacaryocytes et/ou des CTC, éventuellement dans lequel l'enrichissement, l'isolement et/ou la numération se font par tri basé sur la taille et/ou la déformabilité des mégacaryocytes et/ou des CTC.

**10.** Procédé selon une quelconque revendication précédente dans lequel le cancer de la prostate a progressé après que le sujet a subi un traitement pour le cancer de la prostate.

**11.** Procédé selon la revendication 1, dans lequel le cancer de la prostate est un cancer de la prostate localisé, un cancer de la prostate avancé, un cancer de la prostate malin, un cancer de la prostate agressif, un cancer de la prostate résistant à la castration (CPRC) ou un cancer de la prostate métastatique.

**12.** Procédé selon une quelconque revendication précédente comprenant en outre la mesure de concentration d'antigène prostatique spécifique (PSA) et/ou de score de Gleason dans un ou plusieurs échantillons provenant du sujet et/ou la mesure du nombre de métastases chez le sujet.

**13.** Procédé selon la revendication 11 ou la revendication 12, dans lequel le cancer de la prostate a été traité par privation d'hormone et le cancer de la prostate a une ou plusieurs caractéristiques choisies dans le groupe constitué par une concentration de PSA accrue, la taille et le nombre de métastases pendant le traitement malgré une concentration sérique de testostérone inférieure à 50 ng/ml, une montée de PSA supérieure ou égale à 25 % par rapport au nadir et des augmentations consécutives de PSA dans des mesures prises à au moins trois semaines d'écart.

**14.** Procédé selon une quelconque revendication précédente comprenant en outre la mesure d'altération génomique dans les mégacaryocytes et/ou les CTC, dans lequel la mesure d'altération génomique confirme l'identité des mégacaryocytes et/ou des CTC, détermine que les mégacaryocytes sont polyploïdes ou identifie une ou plusieurs mutations des CTC et/ou confirme la malignité des CTC, et comprenant en outre éventuellement la comparaison de l'altération génomique dans les CTC de l'échantillon provenant du sujet avec l'altération génomique dans les CTC d'un témoin de référence, éventuellement dans lequel le témoin de référence est un échantillon provenant d'un individu en bonne santé.

**15.** Procédé selon la revendication 14, dans lequel la mesure d'altération génomique se fait par hybridation *in situ* en fluorescence (FISH), éventuellement dans lequel la FISH est une FISH répétée et éventuellement en outre dans lequel la FISH comprend une dissociation à 40 à 90 °C avec un tampon de dissociation comprenant 1 % à 3 % de dodécylsulfate de sodium (SDS), 0,055 M à 0,075 M de Tris-HCl et 0,5 % à 1,1 % de β-mercaptoéthanol et dans lequel le tampon de dissociation a un pH de 6,6 à 7,0.

Fig. 1

Fig. 2

Fig. 3

44

Fig. 4

Fig. 5

## DAPI

## Fig. 6

17.5 ± 4.9 μm
(range: 10-32)

6.5 ± 0.8 μm
(range: 4-8)

## Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2790020 A
- WO 2004113877 A
- WO 20040144651 A
- US 5837115 A
- US 6692952 B
- WO 2004029221 A
- US 32397105
- US 22790405
- US 9209965 W
- WO 9413804 A

**Non-patent literature cited in the description**

- **SCHER et al.** *Lancet Oncol.,* 2009, vol. 10, 233-239
- **XU et al.** *PLoS One,* 2015, vol. 10, e0138032
- **LEVERSHA et al.** *Clin. Cancer Res.,* 2009, vol. 15 (6), 2091-2097
- **HUME et al.** *N. Engl. J. Med.,* 1964, vol. 270, 111-7
- **LEVERSHA et al.** *Clin Cancer Res,* 2009, vol. 15 (6), 2091-2097
- **LI et al.** *Journal of Bone and Mineral Research,* 2011, vol. 26 (1), 125-134
- **HELLER et al.** *Clinical Cancer Research,* 2016, vol. 23, 1967-1973
- **GUETTA, EM et al.** *Stem Cells Dev,* 2004, vol. 13 (1), 93-9
- **EPSTEIN JI ; ALLSBROOK WC JR ; AMIN MB ; EGEVAD LL.** ISUP Grading Committee. The 2005 International Society of Urological Pathology (ISUP) Consensus Conference on Gleason grading of prostatic carcinoma. *Am J Surg Pathol,* 2005, vol. 29 (9), 1228-42
- **GLEASON DF ; MELLINGER GT.** *J Urol,* 1974, vol. 111, 58-64
- **D'AMICO et al.** *JAMA,* 1998, vol. 280, 969-74
- **NAKAMURA et al.** *Cell Stem Cell,* 2014, vol. 14 (4), 535-548
- **WARD, E.S. et al.** *Nature,* 1989, vol. 341, 544-546
- **BIRD et al.** *Science,* 1988, vol. 242, 423-426
- **HUSTON et al.** *PNAS USA,* 1988, vol. 85, 5879-5883
- **P. HOLLINGER et al.** *Proc. Natl. Acad. Sci. USA,* 1993, vol. 90, 6444-6448
- **HOLLINGER ; WINTER.** *Current Opinion Biotechnol.,* 1993, vol. 4, 446-449
- **TRAUNECKER et al.** *EMBO Journal,* 1991, vol. 10, 3655-3659
- Current Protocols in Molecular Biology. John Wiley & Sons, 1991, 6.3.1-6.3.6
- **EGHOLM et al.** *Peptide Nucleic Acids (PNA)-Oligonucleotide Analogues with an Achiral Peptide Backbone,* 1992
- **BENJAMINI ; HOCHBERG.** *J R Stat Soc Series B Methodol,* 1995, vol. 57, 289-300
- **PSAILA et al.** *J Thromb Haemost,* 2012, vol. 10, 177-188
- **BOJKO et al.** *J Clin Apher,* 1998, vol. 13, 7-15
- **ZIMMET et al.** *Exp Hematol,* 2000, vol. 28, 3-16
- **WINKELMANN et al.** *Haemostasis,* 1984, vol. 14, 501-507
- **ZASLAVSKY et al.** *Blood,* 2010, vol. 115, 4605-4613
- **OGLE et al.** *J Hepatol,* 2016